# EUROPÄISCHE PATENTSCHRIFT

(11) **EP 0 334 911 B1**
(45) Veröffentlichungstag und Bekanntmachung des Hinweises auf die Patenterteilung: **10.02.1993**
(21) Anmeldenummer: 88907653.5
(22) Anmeldetag: 05.09.1988
(51) Int. Cl.: C07C 43/225, C07C 69/88, C07C 69/75, C07D 319/06, C07D 239/26, C07D 239/34, C07D 213/30, C07D 213/643, C09K 19/12, C09K 19/20, C09K 19/34

(54) **SUBSTITUIERTE PHENYLTRIFLUORMETHYLETHER**
SUBSTITUTED PHENYLTRIFLUORMETHYLETHERS
PHENYLTRIFLUOROMETHYLETHERS SUBSTITUES

(30) Priorität: 25.09.1987 DE 3732284
(43) Veröffentlichungstag der Anmeldung: 04.10.1989
(73) Patentinhaber: MERCK PATENT GmbH, D-64271 Darmstadt (DE)
(72) Erfinder: KURMEIER, Hans-Adolf, D-6104 Seeheim-Jugenheim (DE); SCHEUBLE, Bernhard, Yokohama-shi Kanagawa 231 (JP); POETSCH, Eike, D-6109 Mühltal (DE); FINKENZELLER, Ulrich, D-6831 Plankstadt (DE)
(86) Internationale Anmeldenummer: EP8800804
(87) Internationale Veröffentlichungsnummer: WO8902884

(56) Entgegenhaltungen:
- DE-A- 3 511 111
- Mol. Cryst. Liq. Cryst., Band 47, Nr. 1/2, 1978, Gordon and Breach Science Publishers, Ltd., NL; V. V. TITOV et al: "Synthesis and mesomorphism of aryl p-fluoralkyl(alkoxy) benzoates", S. 1-5

## Beschreibung

Die Erfindung betrifft substituierte Phenyltrifluormethylether der Formel I

R¹-(A¹-Z¹)ₙ-A²-Z²-A³-OCF₃ I

worin
- R¹: H oder Alkyl mit 1-18 C-Atomen, worin auch eine oder mehrere CH₂-Gruppen ersetzt sein können durch -E-, -O-, -S- und/oder -CO-, wobei zwei Heteroatome nicht direkt miteinander verknüpft sind, oder Perfluoralkyl mit 1-18 C-Atomen, worin auch eine oder mehrere CF₂-Gruppen ersetzt sein können durch -CH₂-, -E-, -O-, -S- und/oder -CO-, wobei zwei Heteroatome nicht direkt miteinander verknüpft sind,
- E: 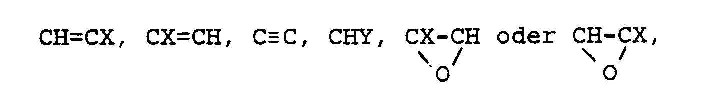
- X: Y, CH₃, H,
- Y: CN, NCS, NCO, Halogen,
- A¹ und A²: jeweils unabhängig voneinander unsubstituiertes oder ein- oder mehrfach durch Halogenatome und/oder CN- und/oder CH₃-Grupen substituiertes 1,4-Phenylen, worin auch eine oder mehrere CH-Gruppen durch N ersetzt sein können, 1,4-Cyclohexylen, worin auch eine oder zwei nicht benachbarte CH₂-Gruppen durch -O- und/oder S-Atome ersetzt sein können, 1,4-Cyclohexenylen, 1,4-Bicyclo(2.2.2)octylen oder Piperidin-1,4-diyl,
- Z¹: -O-CO-, -CH₂O-, -OCH₂-, -CH₂CH₂-, -CH=CH- oder eine Einfachbindung,
- Z²: -CO-O-, -CH₂O-, -OCH₂-, -CH₂CH₂-, -CH=CH- oder eine Einfachbindung,
- A³: eine unsubstituierte oder ein- oder mehrfach durch Halogenatome und/oder CN- und/oder CH₃-Gruppen substituierte 1,4-Phenylengruppe,
und
- n: 0, 1 oder 2
bedeutet, mit den Maßgaben, daß
a) im Falle n = 0 oder 1, R¹ = Alkoxy und A¹ = A² = A³ = unsubstituiertes 1,4-Phenylen, Z² -CH₂O-, -OCH₂-, -CH₂CH₂-, -CH=CH- oder eine Einfachbindung bedeutet, und
b) 4-Phenyltrifluormethoxybenzol ausgenommen ist,
sowie deren Verwendung als Komponenten flüssigkristalliner Phasen.

Der Einfachheit halber bedeuten im folgenden Cyc eine 1,4-Cyclohexylengruppe, Che eine 1,4-Cyclohexenylengruppe, Dio eine 1,3-Dioxan-2,5-diylgruppe, Dit eine 1,3-Dithian-2,5-diylgruppe, Phe eine 1,4-Phenylengruppe, Pyd eine Pyridin-2,5-diylgruppe, Pyr eine Pyrimidin-2,5-diylgruppe und Bi eine Bicyclo(2,2,2)-octylengruppe, wobei Cyc und/oder Phe unsubstituiert oder durch ein oder mehrere Halogenatome und/oder CH₃-Gruppen und/oder CN-Gruppen substituiert sein können.

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Ähnliche Verbindungen sind bereits bekannt: Von V.V. Titov et al. (Mol. Cryst. Liq. Cryst., 47, 1/2, 1978, 1-5) wurden die mesogenen Eigenschaften von p-Trifluormethoxybenzoesäureestern untersucht. Diese sollen jedoch die Tendenz zur Ausbildung smektischer Phasen aufweisen und besitzen zudem relativ niedrige Werte der dielektrischen Anisotropie.

In der DE-OS 35 11 111 werden Verbindungen der Formel 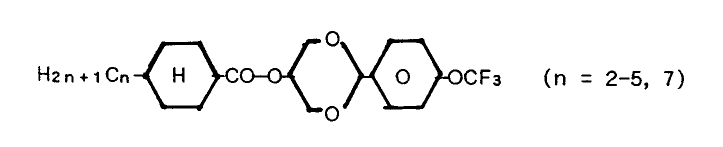
beschrieben. Diese Verbindungen weisen jedoch keine flüssigkristallinen Phasen auf.

In dem U.S. Patent No. 4,157,344 wird die Herstellung von 4-Phenyltrifluormethoxybenzol beschrieben, es findet sich dort jedoch keinerlei Hinweis, daß solche Trifluormethoxybenzolderivate überhaupt als Komponenten flüssigkristalliner Phasen geeignet sind.

Der Erfindung lag die Aufgabe zugrunde, neue stabile flüssigkristalline oder mesogene Verbindungen aufzufinden, die als Komponenten flüssigkristalliner Phasen geeignet sind und insbesondere eine vergleichsweise geringe Viskosität besitzen sowie eine relativ hohe dielektrische Anisotropie.

Es wurde nun gefunden, daß Verbindungen der Formel I als Komponenten flüssigkristalliner Phasen vorzüglich geeignet sind. Insbesondere verfügen sie über vergleichsweise niedere Viskositäten. Mit ihrer Hilfe lassen sich stabile flüssigkristalline Phasen mit breitem Mesophasenbereich und vorteilhaften Werten für die optische und dielektrische Anisotropie erhalten.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektroopische Anzeigeelemente, die derartige Phasen enthalten.

Vor- und nachstehend haben R¹, A¹, Z¹, n, A², Z² und A³ die angegebene Bedeutung, sofern nicht ausdrücklich etwas anderes vermerkt ist.

Die Verbindungen der Formel I umfassen dementsprechend Verbindungen mit zwei Ringen der Teilformeln Ia und Ib:

R¹-A²-A³-OCF₃ Ia

R¹-A²-Z²-A³-OCF₃ Ib

Verbindungen mit drei Ringen der Teilformeln Ic bis If:

R¹-A¹-A²-A³-OCF₃ Ic

R¹-A¹-Z¹-A²-Z²-A³-OCF₃ Id

R¹-A¹-Z¹A²-A³-OCF₃ Ie

R¹-A¹-A²-Z²-A³-OCF₃ If

sowie Verbindungen mit vier Ringen der Teilformeln Ig bis In:

R¹-A¹-A¹-A²-A³-OCF₃ Ig

R¹-A¹-Z¹-A¹-A²-A³-OCF₃ Ih

R¹-A¹-A¹-Z¹-A²-A³-OCF₃ Ii

R¹-A¹-A¹-A²-Z²-A³-OCF₃ ij

R¹-A¹-Z¹-A¹-Z¹-A²-A³-OCF₃ Ik

R¹-A¹-Z¹A¹-A²-Z²-A³-OCF₃ Il

R¹-A¹-A¹-Z¹-A²-Z²-A³-OCF₃ Im

R¹-A¹-Z¹-A¹-Z¹-A²-Z²-A³-OCF₃ In

Darunter sind besonders diejenigen der Teilformeln Ia, Ib, Ic, Id, Ie, If, Ih, Ij und Il bevorzugt.

Die bevorzugten Verbindungen der Teilformel Ia umfassen diejenigen der Teilformeln Iaa bis Iaf:

R¹-Phe-Phe-OCF₃ Iaa

R¹-Dio-Phe-OCF₃ Iab

R¹-Pyr-Phe-OCF₃ Iac

R¹-Pyd-Phe-OCF₃ Iad

R¹-Cyc-Phe-OCF₃ Iae

R¹-Che-Phe-OCF₃ Iaf

Darunter sind diejenigen der Formeln Iaa, Iab, Iad und Iae besonders bevorzugt.

Die bevorzugten Verbindungen der Teilformel Ib umfassen diejenigen der Teilformeln Iba bis Ibl:

R¹-Phe-CH₂CH₂-Phe-OCF₃ Iba

R¹-Phe-OCH₂-Phe-OCF₃ Ibb

R¹-Cyc-CH₂CH₂-Phe-OCF₃ Ibc

R¹-A²-CH₂CH₂-Phe-OCF₃ Ibe

R¹-A²-CH₂O-Phe-OCF₃ Ibf

R¹-A²-OCH₂-Phe-OCF₃ Ibg

R¹-A²-COO-Phe-OCF₃ Ibh

R¹-Che-CH₂CH₂-Phe-OCF₃ Ibj

R¹-Cyc-COO-Phe-OCF₃ Ibl

Die bevorzugten Verbindungen der Teilformel Ic umfassen diejenigen der Teilformeln Ica bis Ici:

R¹-Phe-Phe-Phe-OCF₃ Ica

R¹-Phe-Dio-Phe-OCF₃ Icb

R¹-Cyc-Cyc-Phe-OCF₃ Icc

R¹-Pyd-Phe-Phe-OCF₃ Icd

R¹-Pyr-Phe-Phe-OCF₃ Ice

R¹-Cyc-Phe-Phe-OCF₃ Icf

R¹-Dio-Phe-Phe-OCF₃ Icg

R¹-Che-Phe-Phe-OCF₃ Ich

R¹-Phe-Che-Phe-OCF₃ Ici

Darunter sind diejenigen der Formeln Icc und Icf besonders bevorzugt.

Die bevorzugten Verbindungen der Teilformel Id umfassen diejenigen der Teilformeln Ida bis Idk;

R¹-Phe-Z¹-Phe-Z²-Phe-OCF₃ Ida

R¹-Phe-Z¹-Dio-Z²-Phe-OCF₃ Idb

R¹-Cyc-Z¹-Cyc-Z²-Phe-OCF₃ Idc

R¹-Pyd-Z¹-Phe-Z²-Phe-OCF₃ Ide

R¹-Phe-Z¹-Pyd-Z²-Phe-OCF₃ Idf

R¹-Pyr-Z¹-Phe-Z²-Phe-OCF₃ Idg

R¹-Phe-Z¹-Pyr-Z²-Phe-OCF₃ Idh

R¹-Phe-Z¹-Cyc-Z²-Phe-OCF₃ Idi

R¹-Dio-Z¹-Phe-Z²-Phe-OCF₃ Idj

R¹-Che-Z¹-Phe-Z²-Phe-OCF₃ Idk

Die bevorzugten Verbindungen der Teilformel Ie umfassen diejenigen der Teilformeln Iea bis Iei:

R¹-Pyr-Z¹-Phe-Phe-OCF₃ Iea

R¹-Dio-Z¹-Phe-Phe-OCF₃ Ieb

R¹-Cyc-Z¹-Phe-Phe-OCF₃ Iec

R¹-Phe-Z¹-Cyc-Phe-OCF₃ Ied

R¹-Cyc-Z¹-Cyc-Phe-OCF₃ Iee

R¹-Phe-Z¹-Dio-Phe-OCF₃ Ief

R¹-Pyd-Z¹-Phe-Phe-OCF₃ Ieg

R¹-Phe-Z¹-Pyr-Phe-OCF₃ Ieh

R¹-Phe-Z¹-Che-Phe-OCF₃ Iei

Die bevorzugten Verbindungen der Teilformel If umfassen diejenigen der Teilformeln Ifa bis Ifm:

R¹-Pyr-Phe-Z²-Phe-OCF₃ Ifa

R¹-Pyr-Phe-OCH₂-Phe-OCF₃ Ifb

R¹-Phe-Phe-Z²-Phe-OCF₃ Ifc

R¹-Cyc-Cyc-Z²-Phe-OCF₃ Ifd

R¹-Cyc-Cyc-CH₂CH₂-Phe-OCF₃ Ife

R¹-Pyd-Phe-Z²-Phe-OCF₃ Iff

R¹-Dio-Phe-Z²-Phe-OCF₃ Ifg

R¹-Phe-Cyc-Z²-Phe-OCF₃ Ifh

R¹-Phe-Pyd-Z²-Phe-OCF₃ Ifi

R¹-Che-Phe-Z²-Phe-OCF₃ Ifj

R¹-Phe-Che-Z²-Phe-OCF₃ Ifk

R¹-Pyr-Phe-CH₂CH₂-Phe-OCF₃ Ifl

R¹-Cyc-Phe-CH₂CH₂-Phe-OCF₃ Ifm

Die bevorzugten Verbindungen der Teilformeln Ig bis In umfassen diejenigen der Teilformeln Io bis Iv:

R¹-Cyc-Cyc-Phe-Phe-OCF₃ Io

R¹-A¹-CH₂O-A¹-A²-Phe-OCF₃ Ip

R¹-Cyc-Cyc-Z¹-A²-Phe-OCF₃ Iq

R¹-A¹-A¹-A²-CH₂CH₂-Phe-OCF₃ Ir

R¹-Phe-Z¹-Phe-Z¹-Dio-Phe-OCF₃ Is

R¹-Phe-Z¹-Phe-Phe-Z²-Phe-OCF₃ It

R¹-A¹-A¹-COO-A²-Z²-Phe-OCF₃ Iv

In den Verbindungen der vor- und nachstehenden Formeln bedeutet R¹ vorzugsweise Alkyl, ferner Alkoxy. A¹ und A² sind bevorzugt Phe, Cyc, Che, Pyd, Pyr oder Dio. Bevorzugt enthalten die Verbindungen der Formel I nicht mehr als einen der Reste Bi, Pyd, Pyr, Dio oder Dit.

R¹ stellt vorzugsweise auch eine Alkylgruppe dar, in welcher zwei benachbarte CH₂-Gruppen durch -O- und -CO- oder -CO- und -O- ersetzt sind. Ferner ist R¹ bevorzugt eine Alkylgruppe, in der eine CH₂-Gruppe durch -C≡C- oder -CH=CH- ersetzt ist.

Bevorzugt sind auch Verbindungen der Formel I sowie aller Teilformeln, in denen A¹ und/oder A² durch ein oder mehrere F-Atome substituiertes 1,4-Phenylen ist (sind).

Z¹ und Z² sind bevorzugt Einfachbindungen, in zweiter Linie bevorzugt -CH₂CH₂-, -CH₂O- oder -OCH₂-. Weiterhin sind die Gruppen -COO-, -OCO- oder -CH=CH- für Z¹ und Z² bevorzugt.

A³ ist vorzugsweise eine unsubstituierte oder eine durch ein oder zwei F-Atome substituierte 1,4-Phenylengruppe.

Folgende Verbindungen der Formel 1 bis 13 sind von den lateral substituierten Verbindungen besonders bevorzugt:

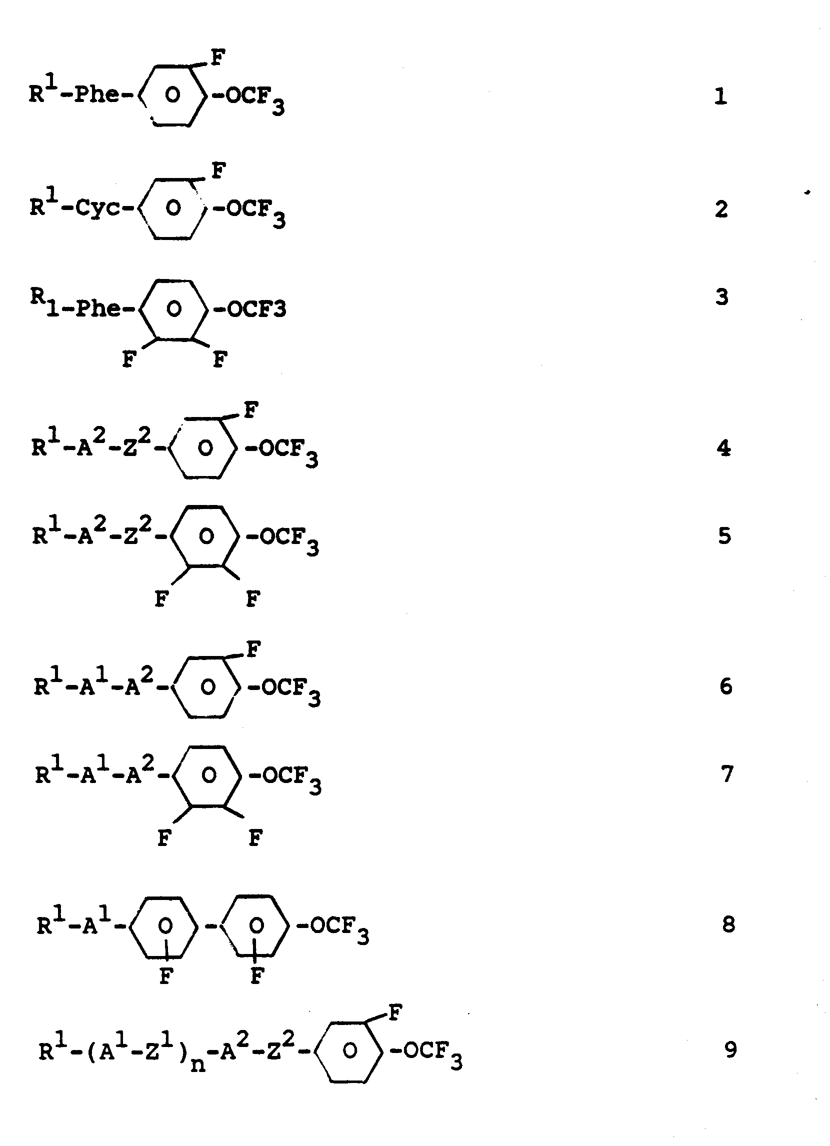

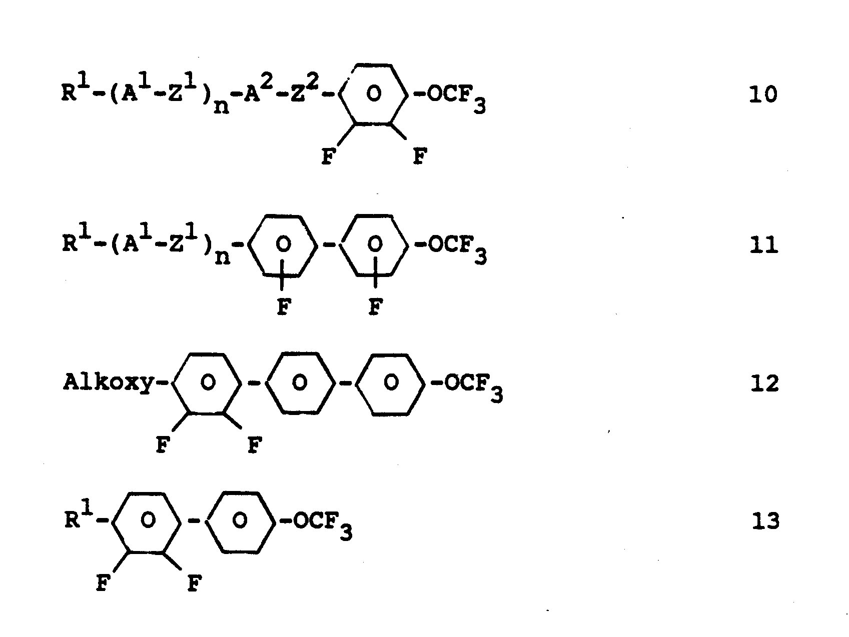

Falls R¹ einen Alkylrest oder einen Alkoxyrest bedeutet, so kann dieser geradkettig oder verzweigt sein. Vorzugsweise ist er geradkettig, hat 2,3,4,5,6,7,8 oder 9-C-Atome und bedeutet demnach bevorzugt Ethyl, Propyl, Butyl, Pentyl, Hexyl, Heptyl, Octyl, Nonyl, Ethoxy, Propoxy, Butyloxy, Pentyloxy, Hexyloxy, Heptyloxy, Octyloxy oder Nonyloxy, ferner auch Methyl, Methoxy, Decyl, Undecyl, Dodecyl, Tridecyl, Tetradecyl, Decyloxy, Undecyloxy, Tridecyloxy oder Tetradecyloxy.

Oxaalkyl bedeutet vorzugsweise geradkettiges 2-Oxapropyl (= Methoxymethyl), 2-(= Ethoxymethyl) oder 3-Oxabutyl (= 2-Methoxyethyl), 2-, 3- oder 4-Oxapentyl, 2-, 3-, 4-oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7-oder 8-Oxanonyl oder 2-, 3-, 4-, 5-, 6-, 7-, 8- oder 9-Oxadecyl.

Falls R¹ einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach bevorzugt Vinyl, Prop-1- oder Prop-2-enyl, But-1-, 2-oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6-oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Verbindungen der Formel I mit verzweigter Flügelgruppe R¹ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verbindungen der Formel I mit S_{A}-Phasen eignen sich für thermisch adressierte Displays.

Verzweigte Gruppen dieser Art enthalten in der Regel nicht mehr als eine Kettenverzweigung. Bevorzugte verzweigte Reste R¹ sind Isopropyl, 2-Butyl (= 1-Methylpropyl), Isobutyl (=2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 2-Octyloxy, 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctanoyloxy, 5-Methylheptyloxycarbonyl, 2-Methylbutyryloxy, 3-Methylvaleryloxy, 4-Methylhexanoyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxa-pentyl, 2-Methyl-3-oxahexyl.

Formel I umfaßt sowohl die Racemate dieser Verbindungen als auch die optischen Antipoden sowie deren Gemische.

R¹ in Formel I bedeutet ferner bevorzugt auch Perfluoralkyl mit vorzugsweise 2, 3, 4, 5, 6, 7, 8 oder 9 C-Atomen.

Unter diesen Verbindungen der Formel I sowie den Unterformeln sind diejenigen bevorzugt, in denen mindestens einer der darin enthaltenen Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Die 1,4-Cyclohexenylen-Gruppe hat vorzugsweise folgende Strukturen:

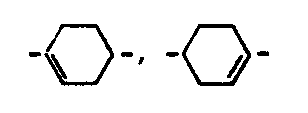

Die Verbindungen der Formel I werden nach an sich bekannten Methoden dargestellt, wie sie in der Literatur (z. B. in den Standardwerken wie Houben-Weyl Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanringes einen Cyclohexenring oder Cyclohexanonring und/oder an Stelle einer -CH₂CH₂-Gruppe eine -CH=CH-Gruppe und/oder an Stelle einer -CH₂-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. PtO₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder -CH₂CH₂-Brücken enthalten, reduziert werden.

Weiterhin sind Reduktionen mit komplexen Hydriden möglich. Beispielsweise können Arylsulfonyloxygruppen mit LiAlH₄ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit NaBH₄ oder Tributylzinnhydrid in Methanol hydriert werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan.

Zur Herstellung von Nitrilen der Formel I (worin A¹, A² und/oder A³ durch mindestens eine CN-Gruppe substituiert ist) können entsprechende Säureamide, z.B. solche, in denen an Stelle des Restes CN eine CONH₂-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie SOCl₂, PCl₃, PCl₅, POCl₃, SO₂Cl₂, COCl₂, ferner P₂O₅, P₂S₅, AlCl₃ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I (worin R eine Alkoxygruppe bedeutet und/oder worin Z¹ und/oder Z² eine -OCH₂- oder eine -CH₂O-Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Zur Herstellung von Nitrilen der Formel I (worin A¹, A² und/oder A³ durch mindestens eine CN-Gruppe substituiert ist) können auch entsprechende Chlor- oder Bromverbindungen der Formel I (worin A¹ und/oder A² und/oder A³ durch mindestens ein Cl- oder Br-Atom substituiert ist) mit einem Cyanid umgesetzt werden, zweckmäßig mit einem Metallcyanid wie NaCN, KCN oder Cu₂(CN)₂, z.B. in Gegenwart von Pyridin in einem inerten Lösungsmittel wie DMF oder N-Methylpyrrolidon bei Temperaturen zwischen 20° und 200°.

Verbindungen der Formel I, worin A¹, A² und/oder A³ durch mindestens ein F- oder Cl-Atom und/oder eine CN-Gruppe substituiert ist, können auch aus den entsprechenden Diazoniumsalzen durch Austausch der Diazoniumgruppe gegen ein Fluor- oder Chloratom oder gegen eine CN-Gruppe, z.B. nach den Methoden von Schiemann oder Sandmeyer, erhalten werden.

Dioxanderivate bzw. Dithianderivate der Formel I (worin eine der Gruppen A¹ und/oder A² eine 1,3-Dioxan-2,5-diyl-Gruppe bzw. 1,3-Dithian-2,5-diyl-Gruppe bedeutet) werden zwecksmäßig durch Reaktion eines entsprechenden Aldehyds (oder eines seiner reaktionsfähigen Derivate) mit einem entsprechenden 1,3-Diol (oder einem seiner reaktionsfähigen Derivate) bzw. einem entsprechenden 1,3-Dithiol hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Bei der Darstellung der Verbindungen der Formel I ist es besonders vorteilhaft von Ausgangsverbindungen auszugehen, die die -OCF₃-Gruppe bereits enthalten, z.B. p-Trifluormethoxybenzaldehyd oder 1-Brom-4-trifluormethoxy-benzol. Weitere Herstellungsmöglichkeiten kann der Fachmann aus der genannten Literatur oder aus den Beispielen entnehmen.

Die erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Biscyclohexylbenzole, 4,4′-Biscyclohexylbiphenyle, Phenyl-oder Cyclohexylpyrimidine, Phenyl- oder Cyclohexyldioxane, Phenyl- oder Cyclohexyldithiane, 1,2-Bis-phenylethane, 1,2-Biscyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

R¹-L-G-E-R² II

worin L und E je ein carbo- oder heterocyclisches Ringsystem aus der aus 1,4-disubstituierten Benzol- und Cyclohexanringen, 4,4′-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe, 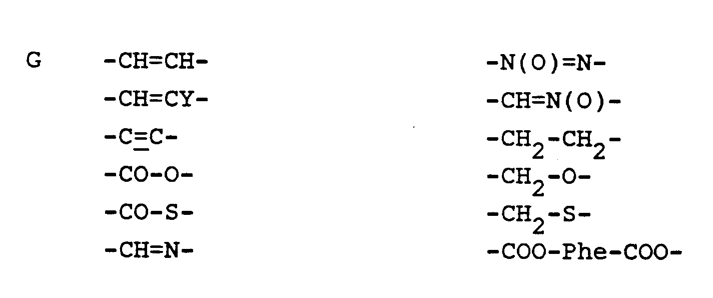
oder eine C-C-Einfachbindung, Y Halogen, vorzugsweise Chlor, oder -CN, und R¹ und R² Alkyl, Alkoxy, Alkanoyloxy oder Alkoxycarbonyloxy mit bis zu 18, vorzugsweise bis zu 8 Kohlenstoffatomen, oder einer dieser Reste auch CN, NO₂, CF₃, F, Cl oder Br bedeuten.

Bei den meisten dieser Verbindungen sind R¹ und R² voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen flüssigkristallinen Phasen enthalten etwa 0,1 bis 99, vorzugsweise 10 bis 95 %, einer oder mehrerer Verbindungen der Formel I. Weiterhin bevorzugt sind flüssigkristalline Phasen, die 0,1 - 50, insbesondere 0,5 - 30 % einer oder mehrerer Verbindungen der Formel I enthalten. Auch isotrope Verbindungen der Formel I können in den erfindungsgemäßen Phasen verwendet werden.

Die Herstellung der erfindungsgemäßen flüssigkristallinen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249 - 258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar.

### Beispiel 1

0,1 mol p-Trifluormethoxybenzaldehyd, 0,1 mol Ethylpropandiol und 0,2 g p-Toluolsulfonsäure werden in 100 ml Toluol 2 Stunden am Rückfluß erhitzt. Nach Eindampfen des Lösungsmittels, Vakuumdestillation und anschließender Kristallisation erhält man Trifluormethoxy-4-(5-ethyl-1,3-dioxan-2-yl)benzol mit Fp. = 31° und Kp. (extr.) = -90°.

Analog werden hergestellt:
Trifluormethoxy-4-(5-propyl-1,3-dioxan-2-yl)benzol, Fp. 36,3°, Kp. 35°
Trifluormethoxy-4-(5-butyl-1,3-dioxan-2-yl)benzol
Trifluormethoxy-4-(5-pentyl-1,3-dioxan-2-yl)benzol, Fp. 23°, Kp. 36°
Trifluormethoxy-4-(5-hexyl-1,3-dioxan-2-yl)benzol
Trifluormethoxy-4-(5-heptyl-1,3-dioxan-2-yl)benzol
Trifluormethoxy-4-(5-octyl-1,3-dioxan-2-yl)benzol
Trifluormethoxy-4-(5-nonyl-1,3-dioxan-2-yl)benzol
Trifluormethoxy-4-(5-decyl-1,3-dioxan-2-yl)benzol
Trifluormethoxy-4-[5-(trans-4-ethylcyclohexyl)-1,3-dioxan-2-yl]benzol
Trifluormethoxy-4-[5-(trans-4-propylcyclohexyl)-1,3-dioxan-2-yl]benzol
Trifluormethoxy-4-[5-(trans-4-butylcyclohexyl)-1,3-dioxan-2-yl]benzol
Trifluormethoxy-4-[5-(trans-4-pentylcyclohexyl)-1,3-dioxan-2-yl]benzol, K 67° S_{B} 146° N 150,5 I
Trifluormethoxy-4-[5-(trans-4-hexylcyclohexyl)-1,3-dioxan-2-yl]benzol
Trifluormethoxy-4-[5-(trans-4-heptylcyclohexyl)-1,3-dioxan-2-yl]benzol

### Beispiel 2

In einem Autoklaven, der auf 0° gekühlt ist, füllt man 2 mol wasserfreie Flußsäure. Dann gibt man eine Mischung aus 0,18 mol Tetrachlormethan und 0,06 mol 4-(trans-4-Ethylcyclohexyl)-phenol zu. Die Mischung wird ca. 8 Stunden bei 150° gerührt, abgekühlt, auf Eiswasser gegossen und mit Ether nachgewaschen. Die beiden Phasen werden ca. 30 Minuten gerührt, getrennt und die Etherlösung mit 5 %iger KOH zur alkalischen Reaktion gewaschen. Nach Trocknen, Abfiltrieren, Abdestillieren und Reinigung erhält man Trifluormethoxy-4-(trans-ethylcyclohexyl)-benzol.

Analog werden hergestellt:
Trifluormethoxy-4-(trans-4-propylcyclohexyl)-benzol
Trifluormethoxy-4-(trans-4-butylcyclohexyl)-benzol
Trifluormethoxy-4-(trans-4-pentylcyclohexyl)-benzol
Trifluormethoxy-4-(trans-4-hexylcyclohexyl)-benzol
Trifluormethoxy-4-(trans-4-heptylcyclohexyl)-benzol
Trifluormethoxy-4-(trans-4-octylcyclohexyl)-benzol
Trifluormethoxy-4-(trans-4-nonylcyclohexyl)-benzol
Trifluormethoxy-4-(trans-4-decylcyclohexyl)-benzol

### Beispiel 3

Zu einem Gemisch aus 8,6 g trans-4-(trans-4-Propylcyclohexyl)cyclohexylbromid und 50 ml THF/Toluol (1:4) gibt man bei 0° 0,42 g Lithium und 3,5 g ZnBr₂. Die Reaktionslösung wird 3 h bei 0°-10° mit Ultraschall behandelt. Nach Zugabe von 7,4 g 1-Brom-4-(trifluormethoxy)-benzol und 0,44 g 1,1-Bis(diphenylphosphino)-ferrocen-palladium(II)-dichlorid [PdCl₂(dppf)] wird 24 Stunden bei Raumtemperatur gerührt, in 25 ml Wasser (+ 5 ml 1 N HCl) gegossen, 15 Min. gerührt, die organische Phase abgetrennt und die wäßrige Phase mit Toluol extrahiert.

Nach Aufarbeitung der organischen Phasen und Reinigung durch Chromatographie und/oder Kristallisation erhält man Trifluormethoxy-4-[trans-4-(trans-4-propyl-cyclohexyl)cyclohexyl]-benzol, K 39° S 68° N 148.6° I.

Analog werden hergestellt:
Trifluormethoxy-4-[trans-4-(trans-4-ethylcyclohexyl)cyclohexyl]-benzol
Trifluormethoxy-4-[trans-4-(trans-4-butylcyclohexyl)cyclohexyl]-benzol
Trifluormethoxy-4-[trans-4-(trans-4-pentylcyclohexyl)cyclohexyl]-benzol
Trifluormethoxy-4-[trans-4-(trans-4-hexylcyclohexyl)cyclohexyl]-benzol
Trifluormethoxy-4-[trans-4-(trans-4-heptylcyclohexyl)cyclohexyl]-benzol
Trifluormethoxy-4-[trans-4-(trans-4-octylcyclohexyl)cyclohexyl]-benzol
Trifluormethoxy-4-[trans-4-(trans-4-nonylcyclohexyl)cyclohexyl]-benzol
Trifluormethoxy-4-[trans-4-(trans-4-decylcyclohexyl)cyclohexyl]-benzol.

### Beispiel 4

Durch Veretherung von 50 mmol Trifluormethoxy-p-hydroxymethylbenzol mit 50 mmol 2-(p-Hydroxybenzol)-5-nonylpyrimidin in 150 ml THF in Gegenwart von 55 mmol Triphenylphosphin und 55 mmol Azodicarbonsäurediethylester erhält man 4-(5-Nonyl-pyrimidin-2-yl)phenyl-(p-trifluormethoxybenzyl)ether mit Fp. = 56° und Kp. = 155°.

Analog werden hergestellt:
4-(5-Ethyl-pyrimidin-2-yl)phenyl-(p-trifluormethoxybenzyl)ether
4-(5-Methyl-pyrimidin-2-yl)phenyl-(p-trifluormethoxybenzyl)ether
4-(5-Propyl-pyrimidin-2-yl)phenyl-(p-trifluormethoxybenzyl)ether
4-(5-Butyl-pyrimidin-2-yl)phenyl-(p-trifluormethoxybenzyl)ether
4-(5-Pentyl-pyrimidin-2-yl)phenyl-(p-trifluormethoxybenzyl)ether
4-(5-Hexyl-pyrimidin-2-yl)phenyl-(p-trifluormethoxybenzyl)ether
4-(5-Heptyl-pyrimidin-2-yl)phenyl-(p-trifluormethoxybenzyl)ether
4-(5-Octyl-pyrimidin-2-yl)phenyl-(p-trifluormethoxybenzyl)ether

### Beispiel 5

Äquimolare Mengen an p-Trifluormethoxy-benzimidamid-Hydrochlorid (herstellbar aus dem entsprechendem Nitril über das entsprechende Benzimidsäureethylester-Hydrochlorid) und Heptylmalondialdehydbisdiethylacetal werden 15 Stunden auf 150° erhitzt. Nach dem Abkühlen wird der Rückstand in Ethanol gelöst. Übliche Aufarbeitung liefert 2-(p-Trifluormethoxybenzol)-5-heptylpyrimidin mit Fp. 23° und Kp 34°.

Analog werden hergestellt:
2-(p-Trifluormethoxybenzol)-5-ethylpyrimidin
2-(p-Trifluormethoxybenzol)-5-propylpyrimidin
2-(p-Trifluormethoxybenzol)-5-butylpyrimidin
2-(p-Trifluormethoxybenzol)-5-pentylpyrimidin
2-(p-Trifluormethoxybenzol)-5-hexylpyrimidin
2-(p-Trifluormethoxybenzol)-5-methoxypyrimidin
2-(p-Trifluormethoxybenzol)-5-ethoxypyrimidin
2-(p-Trifluormethoxybenzol)-5-propoxypyrimidin
2-(p-Trifluormethoxybenzol)-5-butoxypyrimidin
2-(p-Trifluormethoxybenzol)-5-pentyloxypyrimidin
2-(p-Trifluormethoxybenzol)-5-hexyloxypyrimidin
2-(p-Trifluormethoxybenzol)-5-heptyloxypyrimidin

### Beispiel 6

a) Zu einem Gemisch aus 300 g trans-4-(trans-4-Propylcyclohexyl)cyclohexylmethyl-triphenylphosphoniumiodid, 56 g Kalium-tert.butylat und 500 ml THF gibt man bei -10° bis -5° innerhalb 2-3 Stunden 95 g Trifluormethoxybenzaldehyd in 150 ml THF. Man läßt auf Raumtemperatur kommen, neutralisiert mit 2 N HCl, gibt Wasser zu und extrahiert mit Methyl-tert.butyl-ether. Nach Aufarbeitung der organischen Phase und Reinigung durch Chromatographie an Kieselgel erhält man 1-(4-Trifluormethoxyphenyl)-2-[trans-4-(trans-4-propylcyclohexyl)cyclohexyl]-ethen.
b) 162 g des Ethenderivats werden in 800 ml THF über 40 g Pd/C 5 % bei Raumtemperatur und Normaldruck hydriert. Nach Aufarbeitung und Reinigung durch Kristallisation erhält man 1-(4-Trifluormethoxy-phenyl)-2-[trans-4-(trans-4-propylcyclohexy)-cyclohexyl]-ethan mit K 24° K 60° S_{G} 76° N 133.7° I.

Analog werden hergestellt:
1-(4-Trifluormethoxyphenyl)-2-[trans-4-(trans-4-ethyl-cyclohexyl)cyclohexyl]-ethan
1-(4-Trifluormethoxyphenyl)-2-[trans-4-(trans-4-butyl-cyclohexyl)cyclohexyl]-ethan
1-(4-Trifluormethoxyphenyl)-2-[trans-4-(trans-4-pentyl-cyclohexyl)cyclohexyl]-ethan, K 44° S_{B} 108° N 139° I
1-(4-Trifluormethoxyphenyl)-2-[trans-4-(trans-4-hexyl-cyclohexyl)cyclohexyl]-ethan
1-(4-Trifluormethoxyphenyl)-2-[trans-4-(trans-4-heptyl-cyclohexyl)cyclohexyl]-ethan

### Beispiel 7

Zu einem Gemisch aus 1,2 g Magnesium und 25 ml THF gibt man bei Siedetemperatur 15,5 g 1-Brom-4-(trans-4-pentylcyclohexyl)benzol in 50 ml THF. Nach beendeter Zugabe wird 1 Stunde weiter erhitzt, abgekühlt und das Gemisch zu einer Lösung von 6,7 g ZnBr₂ in 50 ml THF bei 0°-15° gegeben. Nach 1 Stunde Rühren werden 12,3 g 1-Brom-4-(trifluormethoxy)benzol und 0,75 g PdCl₂ (dppf) zugegeben. Man rührt 15 Min. bei 5°, anschließend 24 Stunden bei Raumtemperatur. Man gießt auf 100 ml ges. NH₄Cl-Lösung, trennt die organische Phase ab und extrahiert noch mit Toluol. Nach Aufarbeitung der organischen Phase und Reinigung durch Chromatographie und/oder Kristallisation erhält man Trifluormethoxy-4-[4 -(trans-4-pentylcyclohexyl)-phenyl]-benzol, K 43° S_{B} 128° N 147.4° I.

Analog werden hergestellt:
Trifluormethoxy-4-[4-(trans-4-ethylcyclohexyl)phenyl]-benzol
Trifluormethoxy-4-[4-(trans-4-propylcyclohexyl)phenyl]-benzol
Trifluormethoxy-4-[4-(trans-4-butylcyclohexyl)phenyl]-benzol
Trifluormethoxy-4-[4-(trans-4-hexylcyclohexyl)phenyl]-benzol
Trifluormethoxy-4-[4-(trans-4-heptylcyclohexyl)phenyl]-benzol
Trifluormethoxy-4-[4-(trans-4-octylcyclohexyl)phenyl]-benzol
Trifluormethoxy-4-[4-(trans-4-nonylcyclohexyl)phenyl]-benzol
Trifluormethoxy-4-[4-(trans-4-decylcyclohexyl)phenyl]-benzol
Trifluormethoxy-2-fluor-4-[4-(trans-4-ethylcyclohexyl)phenyl]-benzol
Trifluormethoxy-2-fluor-4-[4-(trans-4-propylcyclohexyl)phenyl]-benzol
Trifluormethoxy-2-fluor-4-[4-(trans-4-butylcyclohexyl)phenyl]-benzol
Trifluormethoxy-2-fluor-4-[4-(trans-4-pentylcyclohexyl)phenyl]-benzol
Trifluormethoxy-2-fluor-4-[4-(trans-4-hexylcyclohexyl)phenyl]-benzol
Trifluormethoxy-2-fluor-4-[4-(trans-4-heptylcyclohexyl)phenyl]-benzol
Trifluormethoxy-2-fluor-4-[4-(trans-4-octylcyclohexyl)phenyl]-benzol
Trifluormethoxy-2-fluor-4-[4-(trans-4-nonylcyclohexyl)phenyl]-benzol
Trifluormethoxy-2-fluor-4-[4-(trans-4-decylcyclohexyl)phenyl]-benzol.

### Beispiel 8

Analog Beispiel 7 erhält man aus 4-Brom-4′-pentylbiphenyl und Trifluormethoxy-4-brom-benzol das entsprechende Trifluormethoxy-4-(4′-pentylbiphenyl-4-yl)-benzol.

Analog werden hergestellt:
Trifluormethoxy-4-(4′-ethylbiphenyl-4-yl)-benzol
Trifluormethoxy-4-(4′-propylbiphenyl-4-yl)-benzol
Trifluormethoxy-4-(4′-butylbiphenyl-4-yl)-benzol
Trifluormethoxy-4-(4′-hexylbiphenyl-4-yl)-benzol
Trifluormethoxy-4-(4′-heptylbiphenyl-4-yl)-benzol
Trifluormethoxy-4-(4′-octylbiphenyl-4-yl)-benzol
Trifluormethoxy-2-fluor-4-(4′-ethylbiphenyl-4-yl)-benzol
Trifluormethoxy-2-fluor-4-(4′-propylbiphenyl-4-yl)-benzol
Trifluormethoxy-2-fluor-4-(4′-butylbiphenyl-4-yl)-benzol
Trifluormethoxy-2-fluor-4-(4′-pentylbiphenyl-4-yl)-benzol
Trifluormethoxy-2-fluor-4-(4′-hexylbiphenyl-4-yl)-benzol
Trifluormethoxy-2-fluor-4-(4′-heptylbiphenyl-4-yl)-benzol
Trifluormethoxy-2-fluor-4-(4′-octylbiphenyl-4-yl)-benzol
Trifluormethoxy-4-(2′,3′-difluor-4′-ethoxybiphenyl-4-yl)-benzol
Trifluormethoxy-4-(2′,3′-difluor-4′-propoxybiphenyl-4-yl)-benzol
Trifluormethoxy-4-(2′,3′-difluor-4′-butoxybiphenyl-4-yl)-benzol
Trifluormethoxy-4-(2′,3′-difluor-4′-pentyloxybiphenyl-4-yl)-benzol
Trifluormethoxy-4-(2′,3′-difluor-4′-hexyloxybiphenyl-4-yl)-benzol
Trifluormethoxy-4-(2′,3′-difluor-4′-ethylbiphenyl-4-yl)-benzol
Trifluormethoxy-4-(2′,3′-difluor-4′-propylbiphenyl-4-yl)-benzol
Trifluormethoxy-4-(2′,3′-difluor-4′-butylbiphenyl-4-yl)-benzol
Trifluormethoxy-4-(2′,3′-difluor-4′-pentylbiphenyl-4-yl)-benzol
Trifluormethoxy-4-(2′,3′-difluor-4′-hexylbiphenyl-4-yl)-benzol
Trifluormethoxy-4-(2′,3′-difluor-4′-heptylbiphenyl-4-yl)-benzol

### Beispiel 9

Zu einem Gemisch aus 12 g 2-Ethoxy-5-brompyridin und 50 ml THF gibt man bei -75° 37 ml Butyllithium (15 % in Hexan) und rührt 1 Stunde nach. Anschließend werden 7,5 g ZnBr₂ in 25 ml THF bei -70° bis -65° zugegeben und wiederum 1 Stunde gerührt. Dann werden 14,5 g Trifluormethoxy-4-brombenzol in 25 ml THF und 1 g PdCl₂(dppf) zugefügt. Man läßt die Temperatur auf Raumtemperatur kommen und rührt dann 16 Stunden. Die Aufarbeitung erfolgt analog Beispiel 7 und man erhält nach Reinigung durch Chromatographie und/oder Kristallisation 2-Ethoxy-5-(4-trifluormethoxyphenyl)-pyridin mit K 36°S_{A} ((33°) I.

Analog werden hergestellt:
2-Methoxy-5-(4-trifluormethoxyphenyl)-pyridin
2-Propoxy-5-(4-trifluormethoxyphenyl)-pyridin
2-Butoxy-5-(4-trifluormethoxyphenyl)-pyridin
2-Pentyloxy-5-(4-trifluormethoxyphenyl)-pyridin
2-Hexyloxy-5-(4-trifluormethoxyphenyl)-pyridin
2-Heptyloxy-5-(4-trifluormethoxyphenyl)-pyridin
2-Octyloxy-5-(4-trifluormethoxyphenyl)-pyridin
2-Methyl-5-(4-trifluormethoxyphenyl)-pyridin
2-Ethyl-5-(4-trifluormethoxyphenyl)-pyridin
2-Propyl-5-(4-trifluormethoxyphenyl)-pyridin
2-Butyl-5-(4-trifluormethoxyphenyl)-pyridin
2-Pentyl-5-(4-trifluormethoxyphenyl)-pyridin
2-Hexyl-5-(4-trifluormethoxyphenyl)-pyridin
2-Heptyl-5-(4-trifluormethoxyphenyl)-pyridin
2-Octyl-5-(4-trifluormethoxyphenyl)-pyridin

### Beispiel 10

Analog Beispiel 9 erhält man durch Umsetzung von 14,0 g 2-(4-Bromphenyl)-5-propylpyrimidin und 12,1 g Trifluormethoxy-4-brombenzol das entsprechende 2-(4′-Trifluormethoxybiphenyl-4-yl)-5-propylpyrimidin mit K 125° S_{G} 1110 S_{A} 218° I.

Analog werden hergestellt:
2-(4′-Trifluormethoxybiphenyl-4-yl)-5-methylpyrimidin
2-(4′-Trifluormethoxybiphenyl-4-yl)-5-ethylpyrimidin
2-(4′-Trifluormethoxybiphenyl-4-yl)-5-butylpyrimidin
2-(4′-Trifluormethoxybiphenyl-4-yl)-5-pentylpyrimidin
2-(4′-Trifluormethoxybiphenyl-4-yl)-5-hexylpyrimidin
2-(4′-Trifluormethoxybiphenyl-4-yl)-5-heptylpyrimidin
2-(4′-Trifluormethoxybiphenyl-4-yl)-5-octylpyrimidin
2-(4′-Trifluormethoxybiphenyl-4-yl)-5-methoxypyrimidin
2-(4′-Trifluormethoxybiphenyl-4-yl)-5-ethoxypyrimidin
2-(4′-Trifluormethoxybiphenyl-4-yl)-5-propoxypyrimidin
2-(4′-Trifluormethoxybiphenyl-4-yl)-5-butoxypyrimidin
2-(4′-Trifluormethoxybiphenyl-4-yl)-5-pentyloxypyrimidin
2-(4′-Trifluormethoxybiphenyl-4-yl)-5-hexyloxypyrimidin
2-(4′-Trifluormethoxybiphenyl-4-yl)-5-heptyloxypyrimidin
2-(4′-Trifluormethoxybiphenyl-4-yl)-5-octyloxypyrimidin.

### Beispiel 11

a) Ein Gemisch aus 2,8 g 4-(5-Heptylpyrimidin-2-yl)-styrol, 3,7 g Trifluormethoxy-4-brombenzol, 1,4 ml Triethylamin, 25 ml Acetonitril, 50 mg Pd-II-acetat und 125 mg Tri-o-tolylphosphin wird 36 Stunden am Rückfluß erhitzt. Nach Abkühlen wird eingeengt und man erhält nach Reinigung durch Chromatographie 1-(4-Trifluormethoxyphenyl)-2-[4-(5-heptylpyrimidin-2-yl)phenyl]-ethen mit K 123° S_{c} 151° S_{A} 242° I.
b) Diese Vinylverbindung wird in THF mit Pd (C/5 %) bei Raumtemperatur und Normaldruck hydriert. Nach Aufarbeitung und Reinigung durch Chromatographie und/oder Kristallisation erhält man 1-(4-Trifluormethoxyphenyl)-2-[4-(5-heptylpyrimidin-2-yl)phenyl]-ethan mit K 63° S_{B} 98° S_{A} 144° I.

Analog werden hergestellt:
1-(4-Trifluormethoxyphenyl)-2-[4-(5-ethylpyrimidin-2-yl)-phenyl]-ethan
1-(4-Trifluormethoxyphenyl)-2-[4-(5-propylpyrimidin-2-yl)-phenyl]-ethan
1-(4-Trifluormethoxyphenyl)-2-[4-(5-butylpyrimidin-2-yl)-phenyl]-ethan
1-(4-Trifluormethoxyphenyl)-2-[4-(5-pentylpyrimidin-2-yl)-phenyl]-ethan
1-(4-Trifluormethoxyphenyl)-2-[4-(5-hexylpyrimidin-2-yl)-phenyl]-ethan
1-(4-Trifluormethoxyphenyl)-2-[4-(5-octylpyrimidin-2-yl)-phenyl]-ethan
1-(4-Trifluormethoxyphenyl)-2-[4-(5-ethoxypyrimidin-2-yl)-phenyl]-ethan
1-(4-Trifluormethoxyphenyl)-2-[4-(5-propoxypyrimidin-2-yl)-phenyl]-ethan
1-(4-Trifluormethoxyphenyl)-2-[4-(5-butoxypyrimidin-2-yl)-phenyl]-ethan
1-(4-Trifluormethoxyphenyl)-2-[4-(5-pentyloxypyrimidin-2-yl)-phenyl]-ethan
1-(4-Trifluormethoxyphenyl)-2-[4-(5-hexyloxypyrimidin-2-yl)-phenyl]-ethan
1-(4-Trifluormethoxyphenyl)-2-[4-(5-heptyloxypyrimidin-2-yl)-phenyl]-ethan
1-(4-Trifluormethoxyphenyl)-2-[4-(5-octyloxypyrimidin-2-yl)-phenyl]-ethan

### Beispiel 12

a) Ein Gemisch aus 25,7 g 4-(trans-4-Pentylcyclohexyl)-styrol, 14,5 g 4-(Trifluormethoxy)-iodbenzol, 100 ml Acetonitril, 7 ml Triethylamin, 0,23 g Pd-II-acetat und 0,6 g Tri-o-tolylphosphin wird 24 Stunden auf Siedetemperatur erhitzt. Nach Abkühlen auf 0°, Absaugen der ausgefallenen Substanz, Waschen mit Acetonitril und Wasser, erhält man nach Reinigung durch Kristallisation aus Ethanol/Essigester das 1-(4-Trifluormethoxyphenyl)-2-[4-(trans-4-pentylcyclohexyl)phenyl]-ethen mit K 72° S_{B} 168° S_{A} 194° N 224.1° I.
b) Das Ethenderivat aus a) wird in THF mit Pd-C bei Raumtemperatur und Normaldruck hydriert. Nach Aufarbeitung und Reinigung durch Kristallisation erhält man 1-(4-Trifluormethoxyphenyl)-2-[4-(trans-4-pentylcyclohexyl)phenyl]-ethan.

Analog werden hergestellt:
1-(4-Trifluormethoxyphenyl)-2-[4-(trans-4-methylcyclohexyl)phenyl]-ethan
1-(4-Trifluormethoxyphenyl)-2-[4-(trans-4-ethylcyclohexyl)phenyl]-ethan
1-(4-Trifluormethoxyphenyl)-2-[4-(trans-4-propylcyclohexyl)phenyl]-ethan
1-(4-Trifluormethoxyphenyl)-2-[4-(trans-4-butylcyclohexyl)phenyl]-ethan
1-(4-Trifluormethoxyphenyl)-2-[4-(trans-4-pentylcyclohexyl)phenyl]-ethan
1-(4-Trifluormethoxyphenyl)-2-[4-(trans-4-hexylcyclohexyl)phenyl]-ethan
1-(4-Trifluormethoxyphenyl)-2-[4-(trans-4-heptylcyclohexyl)phenyl]-ethan

### Beispiel 13

Man stellt eine Grignard-Verbindung her aus 1,2 g Magnesium, 50 ml THF und 11,4 g 4-Pentyl-brombenzol in 25 ml THF. Nach beendeter Zugabe erhitzt man noch 1 Stunde unter Rückfluß, kühlt auf 10° ab und gibt 14 g 4-Trifluormethoxy-brombenzol in 25 ml THF und 0,73 g PdCl₂ (dppf) zu.Die Kühlung wird entfernt, die Reaktionsmischung soll aber nicht über 20° steigen. Anschließend rührt man 16 Stunden bei Raumtemperatur, gießt auf 100 ml ges. NH₄Cl-Lösung, rührt noch 15 Minuten und arbeitet dann die organische Phase auf. Nach Reinigung durch Kristallisation erhält man Trifluormethoxy-4-(4-pentylphenyl)benzol mit K 67° I.

Analog werden hergestellt:
Trifluormethoxy-4-(4-methylphenyl)benzol
Trifluormethoxy-4-(4-ethylphenyl)benzol
Trifluormethoxy-4-(4-propylphenyl)benzol
Trifluormethoxy-4-(4-butylphenyl)benzol
Trifluormethoxy-4-(4-hexylphenyl)benzol
Trifluormethoxy-4-(4-heptylphenyl)benzol
Trifluormethoxy-4-(2,3-difluor-4-methylphenyl)benzol
Trifluormethoxy-4-(2,3-difluor-4-ethylphenyl)benzol
Trifluormethoxy-4-(2,3-difluor-4-propylphenyl)benzol
Trifluormethoxy-4-(2,3-difluor-4-butylphenyl)benzol
Trifluormethoxy-4-(2,3-difluor-4-pentylphenyl)benzol
Trifluormethoxy-4-(2,3-difluor-4-ethoxyphenyl)benzol
Trifluormethoxy-4-(2,3-difluor-4-methoxyphenyl)benzol
Trifluormethoxy-4-(2,3-difluor-4-propoxyphenyl)benzol
Trifluormethoxy-4-(2,3-difluor-4-butoxyphenyl)benzol
Trifluormethoxy-4-(2,3-difluor-4-pentyloxyphenyl)benzol

### Beispiel 14

Analog Beispiel 12a) erhält man durch Umsetzung von 7,5 g 4-Ethoxystyrol mit 14,4 g 4-Trifluormethoxyiodbenzol, 6,9 ml Triethylamin, 0,23 g Pd-II-acetat und 0,6 g Tri-o-tolylphosphin in 75 ml Acetonitril das 1-(4-Trifluormethoxyphenyl)-2-(4-ethoxyphenyl)-ethen.

Analog werden hergestellt:
1-(4-Trifluormethoxyphenyl)-2-(4-methoxyphenyl)-ethen
1-(4-Trifluormethoxyphenyl)-2-(4-propoxyphenyl)-ethen
1-(4-Trifluormethoxyphenyl)-2-(4-butoxyphenyl)-ethen
1-(4-Trifluormethoxyphenyl)-2-(4-pentyloxyphenyl)-ethen
1-(4-Trifluormethoxyphenyl)-2-(4-hexyloxyphenyl)-ethen
1-(4-Trifluormethoxyphenyl)-2-(4-heptyloxyphenyl)-ethen
1-(4-Trifluormethoxyphenyl)-2-(4-methylphenyl)-ethen
1-(4-Trifluormethoxyphenyl)-2-(4-ethylphenyl)-ethen
1-(4-Trifluormethoxyphenyl)-2-(4-propylphenyl)-ethen
1-(4-Trifluormethoxyphenyl)-2-(4-butylphenyl)-ethen
1-(4-Trifluormethoxyphenyl)-2-(4-pentylphenyl)-ethen
1-(4-Trifluormethoxyphenyl)-2-(4-hexylphenyl)-ethen
1-(4-Trifluormethoxyphenyl)-2-(4-heptylphenyl)-ethen
1-(4-Trifluormethoxyphenyl)-2-(4-octylphenyl)-ethen

### Beispiel 15

Durch Hydrierung der Ethenderivate, hergestellt in Beispiel 14, mit Pd/C in THF erhält man die entsprechenden Ethanderivative:
1-(4-Trifluormethoxyphenyl)-2-(4-ethoxyphenyl)-ethan
1-(4-Trifluormethoxyphenyl)-2-(4-methoxyphenyl)-ethan
1-(4-Trifluormethoxyphenyl)-2-(4-propoxyphenyl)-ethan
1-(4-Trifluormethoxyphenyl)-2-(4-butoxyphenyl)-ethan
1-(4-Trifluormethoxyphenyl)-2-(4-pentyloxyphenyl)-ethan
1-(4-Trifluormethoxyphenyl)-2-(4-hexyloxyphenyl)-ethan
1-(4-Trifluormethoxyphenyl)-2-(4-heptyloxyphenyl)-ethan
1-(4-Trifluormethoxyphenyl)-2-(4-methylphenyl)-ethan
1-(4-Trifluormethoxyphenyl)-2-(4-ethylphenyl)-ethan
1-(4-Trifluormethoxyphenyl)-2-(4-propylphenyl)-ethan
1-(4-Trifluormethoxyphenyl)-2-(4-butylphenyl)-ethan
1-(4-Trifluormethoxyphenyl)-2-(4-pentylphenyl)-ethan
1-(4-Trifluormethoxyphenyl)-2-(4-hexylphenyl)-ethan
1-(4-Trifluormethoxyphenyl)-2-(4-heptylphenyl)-ethan
1-(4-Trifluormethoxyphenyl)-2-(4-octylphenyl)-ethan.
1-(4-Trifluormethoxyphenyl)-2-(2,3-difluor-4-ethoxyphenyl)-ethan
1-(4-Trifluormethoxyphenyl)-2-(2,3-difluor-4-propoxyphenyl)-ethan
1-(4-Trifluormethoxyphenyl)-2-(2,3-difluor-4-butoxyphenyl)-ethan
1-(4-Trifluormethoxyphenyl)-2-(2,3-difluor-4-pentyloxyphenyl)-ethan
1-(4-Trifluormethoxyphenyl)-2-(2,3-difluor-4-ethylphenyl)-ethan
1-(4-Trifluormethoxyphenyl)-2-(2,3-difluor-4-propylphenyl)-ethan
1-(4-Trifluormethoxyphenyl)-2-(2,3-difluor-4-butylphenyl)-ethan
1-(4-Trifluormethoxyphenyl)-2-(2,3-difluor-4-pentylphenyl)-ethan.

### Beispiel 16

Zu einem Gemisch aus 15,4 g trans-4-Pentylcyclohexylethyliodid und 100 ml THF/Toluol (1:4) werden bei 0° 0,7 g Lithium und 5,7 g ZnBr₂ gegeben, und man behandelt das Reaktionsgemisch bei 0-10° 4 Stunden mit Ultraschall. Anschließend werden bei 5° 14,8 g 4-Trifluormethoxybrombenzol und 0,88 g PdCl₂ (dppf) zugegeben. Man läßt auf Raumtemperatur kommen und rührt noch 16 Stunden. Dann wird auf 100 ml ges. NH₄Cl-Lösung gegossen und die organische Phase aufgearbeitet. Nach Reinigung durch Chromatographie und/oder Kristallisation erhält man 1-(4-Trifluormethoxyphenyl)-2-(trans-4-pentylcyclohexyl)-ethan.

Analog werden hergestellt:
1-(4-Trifluormethoxyphenyl)-2-(trans-4-ethylcyclohexyl)-ethan
1-(4-Trifluormethoxyphenyl)-2-(trans-4-propylcyclohexyl)-ethan
1-(4-Trifluormethoxyphenyl)-2-(trans-4-butylcyclohexyl)-ethan
1-(4-Trifluormethoxyphenyl)-2-(trans-4-hexylcyclohexyl)-ethan
1-(4-Trifluormethoxyphenyl)-2-(trans-4-heptylcyclohexyl)-ethan

### Beispiel 17

Aus 15,4 g trans-4- Propylcyclohexylbromid und 18,2 g 4-Trifluormethoxybrombenzol erhält man analog Beispiel 16 nach Reinigung durch Chromatographie und/oder Vakuumdestillation Trifluormethoxy-4-(trans-4-propylcyclohexyl)benzol.

Analog werden hergestellt:
Trifluormethoxy-4-(trans-4-ethylcyclohexyl)benzol
Trifluormethoxy-4-(trans-4-butylcyclohexyl)benzol
Trifluormethoxy-4-(trans-4-pentylcyclohexyl)benzol
Trifluormethoxy-4-(trans-4-hexylcyclohexyl)benzol
Trifluormethoxy-4-(trans-4-heptylcyclohexyl)benzol
Trifluormethoxy-4-(trans-4-octylcyclohexyl)benzol

### Beispiel 18

Zu einem Gemisch aus 4 g 4-Trifluormethoxyphenol und 20 ml Toluol gibt man zunächst 4,6 g Dicyclohexylcarbodiimid in 80 ml Toluol, fügt dann 0,2 g Dimethylaminopyridin zu, rührt 1 Stunde bei Raumtemperatur und gibt dann 3,8 g trans-4-Ethylcyclohexancarbonsäure zu. Nach Aufarbeitung und Reinigung durch Chromatographie und/oder Kristallisation erhält man trans-4-Ethylcyclohexancarbonsäure-4-(trifluormethoxy)phenylester.

Analog werden hergestellt:
trans-4-Propylcyclohexancarbonsäure-4-(trifluormethoxy)phenylester
trans-4-Butylcyclohexancarbonsäure-4-(trifluormethoxy)phenylester
trans-4-Pentylcyclohexancarbonsäure-4-(trifluormethoxy)phenylester
trans-4-Hexylcyclohexancarbonsäure-4-(trifluormethoxy)phenylester
trans-4-Heptylcyclohexancarbonsäure-4-(trifluormethoxy)phenylester
trans-4-(trans-4-Propylcyclohexyl)cyclohexancarbonsäure-4-(trifluormethoxy)phenylester
trans-4-(trans-4-Ethylcyclohexyl)cyclohexancarbonsäure-4-(trifluormethoxy)phenylester
trans-4-(trans-4-Butylcyclohexyl)cyclohexancarbonsäure-4-(trifluormethoxy)phenylester
trans-4-(trans-4-Pentylcyclohexyl)cyclohexancarbonsäure-4-(trifluormethoxy)phenylester
trans-4-(trans-4-Hexylcyclohexyl)cyclohexancarbonsäure-4-(trifluormethoxy)phenylester
trans-4-(trans-4-Heptylcyclohexyl)cyclohexancarbonsäure-4-(trifluormethoxy)phenylester

Folgende Beispiele betreffen erfindungsgemäße flüssigkristalline Phasen:

### Beispiel A:

Eine flüssigkristalline Phase, bestehend aus

| | |
|---|---|
| 16 % | p-trans-4-Propylcyclohexyl-benzonitril, |
| 4 % | p-trans-4-Pentylcyclohexyl-benzonitril, |
| 9 % | Trifluormethoxy-4-(5-propyl-1,3-dioxan-2-yl)-benzol, |
| 9 % | Trifluormethoxy-4-(5-pentyl-1,3-dioxan-2-yl)-benzol, |
| 20 % | trans-1-p-Methoxyphenyl-4-propylcyclohexan, |
| 11 % | 4-Ethyl-4'-(trans-4-propylcyclohexyl)-biphenyl, |
| 11 % | 4-Ethyl-4'-(trans-4-pentylcyclohexyl)-biphenyl, |
| 4 % | 4,4'-Bis-(trans-4-propylcyclohexyl)-biphenyl, |
| 6 % | 4-(trans-4-Pentylcyclohexyl)-4'-(trans-4-propylcyclohexyl)-biphenyl, |
| 3 % | 4-(trans-4-Propylcyclohexyl)-2'-fluor-4'-(trans-4-propylcyclohexyl)-biphenyl, |
| 4 % | 2-Fluor-4-(trans-4-pentylcyclohexyl)-4′-(trans-4-propylcyclohexyl)-biphenyl |
| | und |
| 3 % | 4-(trans-4-Pentylcyclohexyl)-2′-fluor-4′-(trans-4-pentylcyclohexyl)-biphenyl |

hat einen Schmelzpunkt von -17°, einen Klärpunkt von 92° und eine Viskosität von 21 mm²/s bei 20°.

### Beispiel B

Eine flüssigkristalline Phase, bestehend aus

| | |
|---|---|
| 8 % | p-trans-4-Propylcyclohexyl-benzonitril, |
| 12 % | Trifluormethoxy-4-(5-propyl-1,3-dioxan-2-yl)-benzol, |
| 10 % | p-trans-4-Butylcyclohexyl-benzonitril, |
| 12 % | trans-1-p-Ethylphenyl-4-propylcyclohexan, |
| 6 % | trans-1-p-Methoxyphenyl-4-propylcyclohexan, |
| 13 % | 4-Ethyl-4′-(trans-4-propylcyclohexyl)-biphenyl, |
| 12 % | 4-Ethyl-4′-(trans-4-pentylcyclohexyl)-biphenyl, |
| 3 % | 4,4′-Bis-(trans-4-propylcyclohexyl)-biphenyl, |
| 6 % | 4-(trans-4-Pentylcyclohexyl)-4′-(trans-4-propylcyclohexyl)-biphenyl, |
| 3 % | 4,4′-Bis-(trans-4-pentylcyclohexyl)-biphenyl, |
| 4 % | 4-(trans-4-Pentylcyclohexyl)-4′-(trans-4-propylcyclohexyl)-biphenyl |
| 7 % | 2-Fluor-4-(trans-4-pentylcyclohexyl)-4′-(trans-4-propylcyclohexyl)-biphenyl |
| | und |
| 4 % | 4-(trans-4-Pentylcyclohexyl)-2′-fluor-4′-(trans-4-pentylcyclohexyl)-biphenyl |

hat einen Schmelzpunkt von -20,8°, einen Klärpunkt von 108° und eine Viskosität von 25 mm²/s bei 20°.

### Beispiel C :

Eine flüssigkristalline Phase, bestehend aus

| | |
|---|---|
| 7 % | 2-p-Cyanphenyl-5-propyl-1,3-dioxan |
| 6 % | 2-p-Cyanphenyl-5-butyl-1,3-dioxan, |
| 4 % | 2-p-Cyanphenyl-5-pentyl-1,3-dioxan, |
| 9 % | Trifluormethoxy-4-(5-propyl-1,3-dioxan-2-yl)-benzol, |
| 9 % | Trifluormethoxy-4-(5-pentyl-1,3-dioxan-2-yl)-benzol, |
| 12 % | 4-Ethyl-4′-(trans-4-propylcyclohexyl)-biphenyl, |
| 10 % | 4-Ethyl-4′-(trans-4-pentylcyclohexyl)-biphenyl, |
| 5 % | 2-p-Pentyloxphenyl-5-hexylpyrimidin, |
| 5 % | 2-p-Hexyloxyphenyl-5-hexylprimidin, |
| 5 % | 2-p-Heptyloxyphenyl-5-hexylpyrimidin, |
| 4 % | 2-p-Propoxyphenyl-5-hexylpyrimidin, |
| 4 % | 2-p-Heptyloxyphenyl-5-heptylpyrimidin, |
| 4 % | 2-p-Nonyloxyphenyl-5-heptylpyrimidin, |
| 7 % | 2-Fluor-4-(trans-4-pentylcyclohexyl)-4′-(trans-4-propylcyclohexyl)-biphenyl |
| | und |
| 9 % | trans-1-p-Methoxyphenyl-4-propylcyclohexan |

hat K -17° Sm 30° N 70° I.

### Beispiel D

Eine flüssigkristalline Phase, bestehend aus

| | |
|---|---|
| 20 % | 1-(4-Trifluormethoxyphenyl)-2-[trans-4-(trans-4-propylcyclohexyl)cyclohexyl]-ethan, |
| 20 % | 1-(4-Trifluormethoxyphenyl)-2-[trans-4-(trans-4-pentylcyclohexyl)cyclohexyl]-ethan, |
| 20 % | trans-1-p-Methoxyphenyl-4-propylcyclohexan, |
| 20 % | trans-1-p-Ethoxyphenyl-4-propylcyclohexan |
| | und |
| 20 % | trans-1-p-Butoxyphenyl-4-propylcyclohexan |

hat einen Schmelzpunkt von -6.2°, einen Klärpunkt von 64° und eine Viskosität von 12 mm²/s bei 20°.

### Beispiel E:

Eine flüssigkristalline Phase, bestehend aus

| | |
|---|---|
| 20 % | p-trans-4-Propylcyclohexyl-benzonitril, |
| 20 % | trans-1-p-Propylphenyl-4-pentylcyclohexan, |
| 15 % | trans-1-p-Ethoxyphenyl-4-propylcyclohexan, |
| 15 % | trans-1-p-Butoxyphenyl-4-propylcyclohexan, |
| 15 % | 4-Trifluormethoxy-4′-(trans-4-propylcyclohexyl)-biphenyl |
| | und |
| 15 % | 4-Trifluormethoxy-4′-(trans-4-pentylcyclohexyl)-biphenyl |

hat einen Klärpunkt von 62°, eine Viskosität von 12 mm²/s bei 20° und Δn = +0.12.

### Beispiel F

Eine flüssigkristalline Phase, bestehend aus

| | |
|---|---|
| 10 % | p-trans-4-Propylcyclohexyl-benzonitril, |
| 10 % | 2-p-Cyanphenyl-5-propyl-1,3-dioxan, |
| 10 % | 4-Propyl-4′-cyanbiphenyl, |
| 10 % | 4-Propylbenzoesäure-p-cyanphenylester, |
| 10 % | 4-Propylbenzoesäure-(3-fluor-4-cyanphenylester), |
| 10 % | trans-1-p-Methoxyphenyl-4-propylcyclohexan, |
| 10 % | trans-1-p-Ethoxyphenyl-4-propylcyclohexan, |
| 15 % | 4-Ethyl-1-trans-4-(trans-4-propylcyclohexyl)-cyclohexyl-benzol |
| | und |
| 15 % | 4-Trifluormethoxy-1-trans-4-(trans-4-propylcyclohexyl)cyclohexyl-benzol |

hat einen Klärpunkt von 66°, eine Viskosität von 22 mm²/s und Δn = +0.14.

### Beispiel G

Eine flüssigkristalline Phase, bestehend aus

| | |
|---|---|
| 7 % | 2-p-Cyanphenyl-5-propyl-1,3-dioxan, |
| 6 % | 2-p-Cyanphenyl-5-butyl-1,3-dioxan, |
| 4 % | 2-p-Cyanphenyl-5-pentyl-1,3-dioxan, |
| 9 % | 1-(4-Trifluormethoxyphenyl)-2-[trans-4-(trans-4-propylcyclohexyl)cyclohexyl]-ethan, |
| 9 % | 1-(4-Trifluormethoxyphenyl-2-[trans-4-(trans-4-pentylcyclohexyl)cyclohexyl]-ethan, |
| 12 % | 4-Ethyl-4′-(trans-4-propylcyclohexyl)-biphenyl, |
| 10 % | 4-Ethyl-4′-(trans-4-pentylcyclohexyl)-biphenyl, |
| 5 % | 2-p-Pentyloxyphenyl-5-hexylpyrimidin, |
| 5 % | 2-p-Hexyloxyphenyl-5-hexylpyrimidin, |
| 5 % | 2-p-Heptyloxyphenyl-5-hexylpyrimidin, |
| 4 % | 2-p-Propoxyphenyl-5-hexylpyrimidin, |
| 4 % | 2-p-Heptyloxyphenyl-5-heptylpyrimidin, |
| 4 % | 2-p-Nonyloxyphenyl-5-heptylpyrimidin, |
| 7 % | 2-Fluor-4-(trans-4-pentylcyclohexyl)-4′-(trans-4-propylcyclohexyl)-biphenyl |
| | und |
| 9 % | trans-1-p-Methoxyphenyl-4-propylcyclohexan |

hat einen Schmelzpunkt -17°, einen Klärpunkt von 99° und eine Viskosität von 32 mm²/s bei 20°.

### Beispiel H

Eine flüssigkristalline Phase, bestehend aus

| | |
|---|---|
| 8 % | p-trans-4-Propylcyclohexyl-benzonitril, |
| 12 % | 1-(4-Trifluormethoxyphenyl)-2-[trans-4-(trans-4-propylcyclohexyl)cyclohexyl]-ethan |
| 10 % | p-trans-4-Butylcyclohexyl-benzonitril, |
| 12 % | trans-1-p-Ethylphenyl-4-propylcyclohexan, |
| 6 % | trans-1-p-Methoxyphenyl-4-propylcyclohexan, |
| 13 % | 4-Ethyl-4′-(trans-4-propylcyclohexyl)-biphenyl, |
| 12 % | 4-Ethyl-4′-(trans-4-pentylcyclohexyl)-biphenyl, |
| 3 % | 4,4′-Bis-(trans-4-propylcyclohexyl)-biphenyl, |
| 6 % | 4-(trans-4-Pentylcyclohexyl)-4′-(trans-4-propylcyclohexyl)-biphenyl, |
| 3 % | 4,4′-Bis-(trans-4-pentylcyclohexyl)-biphenyl, |
| 4 % | 4-(trans-4-Pentylcyclohexyl)-4′-(trans-4-propylcyclohexyl)-biphenyl, |
| 7 % | 2-Fluor-4-(trans-4-pentylcyclohexyl)-4′-(trans-4-propylcyclohexyl)-biphenyl |
| | und |
| 4 % | 4-(trans-4-Pentylcyclohexyl)-2′-fluor-4′-(trans-4-pentylcyclohexyl)-biphenyl |

hat einen Schmelzpunkt von -21°, einen Klärpunkt von 123° und eine Viskosität von 27 mm²/s bei 20°.

### Beispiel I

Eine flüssigkristalline Phase, bestehend aus

| | |
|---|---|
| 16 % | p-trans-4-Propylcyclohexyl-benzonitril, |
| 4 % | p-trans-4-Pentylcyclohexyl-benzonitril, |
| 9 % | 1-(4-Trifluormethoxyphenyl)-2-[trans-4-(trans-4-propylcyclohexyl)cyclohexyl]-ethan |
| 9 % | 1-(4-Trifluormethoxyphenyl)-2-[trans-4-(trans-4-pentylcyclohexyl)cyclohexyl]-ethan, |
| 20 % | trans-1-p-Methoxyphenyl-4-propylcyclohexan, |
| 11 % | 4-Ethyl-4′-(trans-4-propylcyclohexyl)-biphenyl, |
| 11 % | 4-Ethyl-4′-(trans-4-pentylcyclohexyl)-biphenyl, |
| 4 % | 4,4′-Bis-(trans-4-propylcyclohexyl)-biphenyl, |
| 6 % | 4-(trans-4-Pentylcyclohexyl)-4′-(trans-4-propylcyclohexyl)-biphenyl, |
| 3 % | 4-(trans-4-Propylcyclohexyl)-2′-fluor-4′-(trans-4-propylcyclohexyl)-biphenyl, |
| 4 % | 2-Fluor-4-(trans-4-pentylcyclohexyl)-4′-(trans-4-propylcyclohexyl)-biphenyl |
| | und |
| 3 % | 4-(trans-4-Pentylcyclohexyl-2′-fluor-4′-(trans-4-pentylcyclohexyl)-biphenyl |

hat einen Schmelzpunkt von -15°, einen Klärpunkt von 120° und eine Viskosität von 23 mm²/s bei 20°.

## Patentansprüche

1. Substituierte Phenyltrifluormethylether der Formel I
R¹-(A¹-Z¹)ₙ-A²-Z²-A³-OCF₃ I
worin
R¹ H oder Alkyl mit 1-18 C-Atomen, worin auch eine oder mehrere CH₂-Gruppen ersetzt sein können durch -E-, -O-, -S- und/oder -CO-, wobei zwei Heteroatome nicht direkt miteinander verknüpft sind, oder Perfluoralkyl mit 1-18 C-Atomen, worin auch eine oder mehrere CF₂-Gruppen ersetzt sein können durch -CH₂-, -E-, -O-, -S- und/oder -CO-, wobei zwei Heteroatome nicht direkt miteinander verknüpft sind,
E 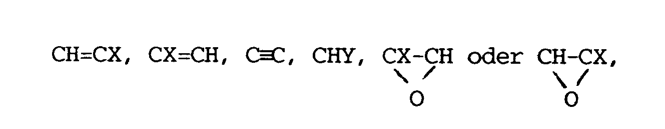
X Y, CH₃, H,
Y CN, NCS, NCO, Halogen,
A¹ und A² jeweils unabhängig voneinander unsubstituiertes oder ein- oder mehrfach durch Halogenatome und/oder CN- und/oder CH₃-Grupen substituiertes 1,4-Phenylen, worin auch eine oder mehrere CH-Gruppen durch N ersetzt sein können, 1,4-Cyclohexylen, worin auch eine oder zwei nicht benachbarte CH₂-Gruppen durch -O- und/oder S-Atome ersetzt sein können, 1,4-Cyclohexenylen, 1,4-Bicyclo(2.2.2)octylen oder Piperidin-1,4-diyl,
Z¹ -O-CO-, -CH₂O-, -OCH₂-, -CH₂CH₂-, -CH=CH- oder eine Einfachbindung,
Z² -CO-O-, -CH₂O-, -OCH₂-, -CH₂CH₂-, -CH=CH- oder eine Einfachbindung,
A³ eine unsubstituierte oder ein- oder mehrfach durch Halogenatome und/oder CN- und/oder CH₃-Gruppen substituierte 1,4-Phenylengruppe,
und
n 0, 1 oder 2
bedeutet, mit den Maßgaben, daß
a) im Falle n = 0 oder 1, R¹ = Alkoxy und A¹ = A² = A³ = unsubstituiertes 1,4-Phenylen, Z² -CH₂O-, -OCH₂-, -CH₂CH₂-, -CH=CH- oder eine Einfachbindung bedeutet, und
b) 4-Phenyltrifluormethoxybenzol ausgenommen ist.

2. Phenyltrifluormethylether nach Anspruch 1 ausgewählt aus den Teilformeln Ia und Ib:
R¹-A²-A³-OCF₃ Ia
R¹-A²-Z²-A³-OCF₃ Ib
worin R¹, A², A³ und Z² die in Anspruch 1 gegebene Bedeutung besitzen.

3. Phenyltrifluormethylether nach Anspruch 1 ausgewählt aus den Teilformeln Ic bis If:
R¹-A¹-A²-A³-OCF₃ Ic
R¹-A¹-Z¹-A²-Z²-A³-OCF₃ Id
R¹-A¹-Z¹-A²-A³-OCF₃ Ie
R¹-A¹-A²-Z²-A³-OCF₃ If
worin R¹, A¹, A², A³, Z¹ und Z² die in Anspruch 1 gegebene Bedeutung besitzen.

4. Phenyltrifluormethylether nach Anspruch 1 ausgewählt aus den Teilformeln Ig bis In:
R¹-A¹-A¹-A²-A³-OCF₃ Ig
R¹-A¹-Z¹-A¹-A²-A³-OCF₃ Ih
R¹-A¹-A¹-Z¹-A²-A³-OCF₃ Ii
R¹-A¹-A¹-A²-Z²-A³-OCF₃ Ij
R¹-A¹-Z¹-A¹-Z¹-A²-A³-OCF₃ Ik
R¹-A¹-Z¹-A¹-A²-Z²-A³-OCF₃ Il
R¹-A¹-A¹-Z¹-A²-Z²-A³-OCF₃ Im
R¹-A¹-Z¹-A¹-Z¹-A²-Z²-A³-OCF₃ In
worin R¹, A¹, A², A³, Z¹ und Z² die in Anspruch 1 gegebene Bedeutung besitzen.

5. Phenyltrifluormethylether nach Anspruch 2, ausgewählt aus den Teilformeln Iaa bis Iaf:
R¹-Phe-Phe-OCF₃ Iaa
R¹-Dio-Phe-OCF₃ Iab
R¹-Pyr-Phe-OCF₃ Iac
R¹-Pyd-Phe-OCF₃ Iad
R¹-Cyc-Phe-OCF₃ Iae
R¹-Che-Phe-OCF₃ Iaf
worin
R¹ die in Anspruch 1 angegebene Bedeutung besitzt,
Phe eine unsubstituierte oder durch ein oder mehrere Halogenatome und/oder CH₃-Gruppen und/oder CN-Gruppen substituierte 1,4-Phenylengruppe,
Dio eine 1,3-Dioxan-2,5-diylgruppe,
Pyr eine Pyrimidin-2,5-diylgruppe,
Pyd eine Pyridin-2,5-diylgruppe,
Cyc eine unsubstituierte oder durch eine oder mehrere Halogenatome und/oder CH₃-Gruppen und/oder CN-Gruppen substituierte 1,4-Cyclohexylengruppe, und
Che eine 1,4-Cyclohexenylengruppe
bedeuten.

6. Phenyltrifluormethylether nach Anspruch 3, ausgewählt aus den Teilformeln Ica bis Ici
R¹-Phe-Phe-Phe-OCF₃ Ica
R¹-Phe-Dio-Phe-OCF₃ Icb
R¹-Cyc-Cyc-Phe-OCF₃ Icc
R¹-Pyd-Phe-Phe-OCF₃ Icd
R¹-Pyr-Phe-Phe-OCF₃ Ice
R¹-Cyc-Phe-Phe-OCF₃ Icf
R¹-Dio-Phe-Phe-OCF₃ Icg
R¹-Che-Phe-Phe-OCF₃ Ich
R¹-Phe-Che-Phe-OCF₃ Ici
worin R¹ die in Anspruch 1 gegebene Bedeutung besitzt, Phe, Dio, Cycl, Pyd, Pyr und Che die in Anspruch 5 gegebene Bedeutung besitzen.

7. Phenyltrifluormethylether nach Anspruch 3, ausgewählt aus den Teilformeln Iea bis Iei:
R¹-Pyr-Z¹-Phe-Phe-OCF₃ Iea
R¹-Dio-Z¹-Phe-Phe-OCF₃ Ieb
R¹-Cyc-Z¹-Phe-Phe-OCF₃ Iec
R¹-Phe-Z¹-Cyc-Phe-OCF₃ Ied
R¹-Cyc-Z¹-Cyc-Phe-OCF₃ Iee
R¹-Phe-Z¹-Dio-Phe-OCF₃ Ief
R¹-Pyd-Z¹-Phe-Phe-OCF₃ Ieg
R¹-Phe-Z¹-Pyr-Phe-OCF₃ Ieh
R¹-Phe-Z¹-Che-Phe-OCF₃ Iei
worin R¹ und Z¹ die in Anspruch 1 gegebene Bedeutung besitzen und Phe, Dio, Cyc, Pyr und Che die in Anspruch 5 gegebene Bedeutung besitzen.

8. Phenyltrifluormethylether nach Anspruch 3, ausgewählt aus den Teilformeln Ifa bis Ifm:
R¹-Pyr-Phe-Z²-Phe-OCF₃ Ifa
R¹-Pyr-Phe-OCH₂-Phe-OCF₃ Ifb
R¹-Phe-Phe-Z²-Phe-OCF₃ Ifc
R¹-Cyc-Cyc-Z²-Phe-OCF₃ Ifd
R¹-Cyc-Cyc-CH₂CH₂-Phe-OCF₃ Ife
R¹-Pyd-Phe-Z²-Phe-OCF₃ Iff
R¹-Dio-Phe-Z²-Phe-OCF₃ Ifg
R¹-Phe-Cyc-Z²-Phe-OCF₃ Ifh
R¹-Phe-Pyd-Z²-Phe-OCF₃ Ifi
R¹-Che-Phe-Z²-Phe-OCF₃ Ifj
R¹-Phe-Che-Z²-Phe-OCF₃ Ifk
worin R¹ und Z¹ die in Anspruch 1 gegebene Bedeutung besitzen und Phe, Dio, Cyc, Pyd, Pyr und Che die in Anspruch 5 gegebene Bedeutung besitzen.

9. Phenyltrifluormethylether nach einem der Ansprüche 1 bis 4 dadurch gekennzeichnet, daß A³ eine unsubstituierte oder eine durch ein oder zwei F-Atome substituierte 1,4-Phenylengruppe ist.

10. Phenyltrifluormethylether nach Anspruch 9, ausgewählt aus den Formeln 1 bis 13:

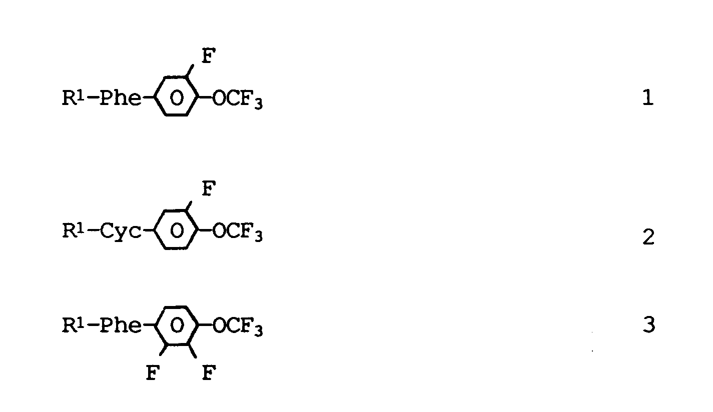

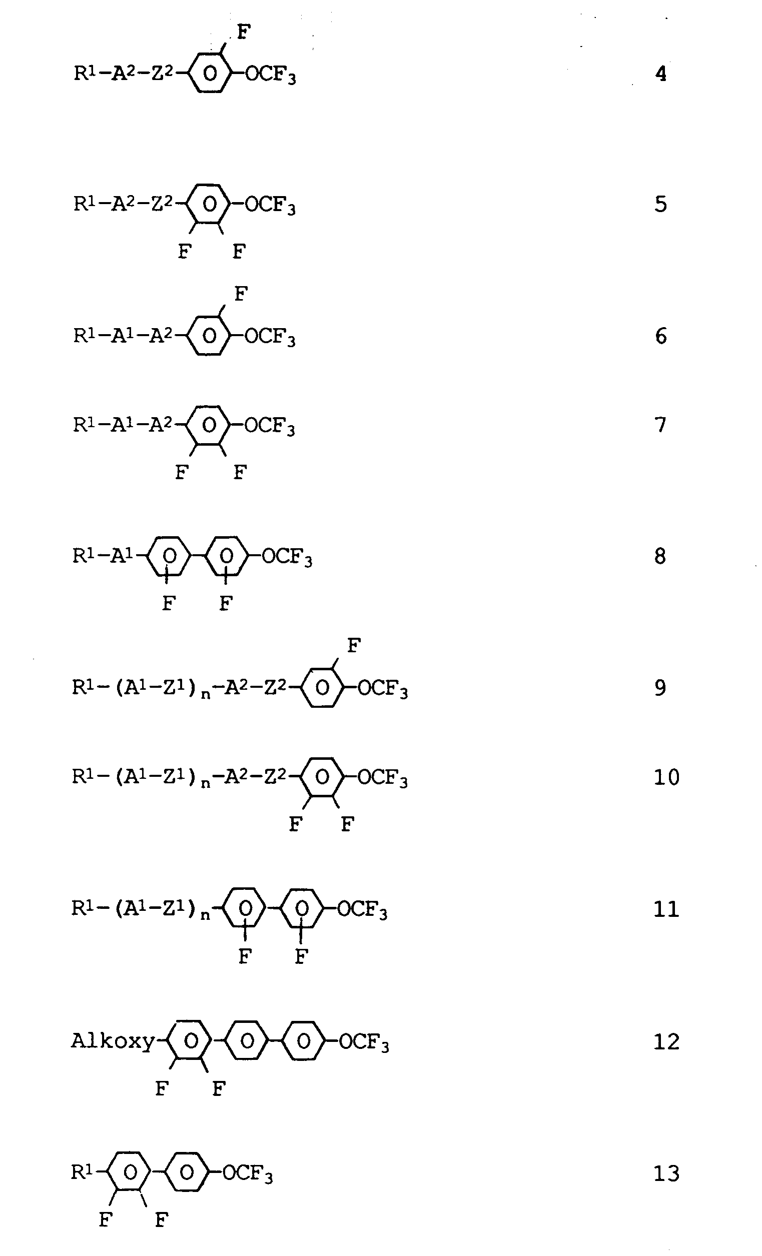

11. Trifluormethylphenylether nach Anspruch 5, ausgewählt aus:
Trifluormethoxy-4-(trans-ethylcyclohexyl)-benzol
Trifluormethoxy-4-(trans-4-propylcyclohexyl)-benzol
Trifluormethoxy-4-(trans-4-butylcyclohexyl)-benzol
Trifluormethoxy-4-(trans-4-pentylcylcohexyl)-benzol
Trifluormethoxy-4-(trans-4-hexylcyclohexyl)-benzol
Trifluormethoxy-4-(trans-4-heptylcyclohexyl)-benzol
Trifluormethoxy-4-(trans-4-octylcyclohexyl)-benzol
Trifluormethoxy-4-(trans-4-nonylcyclohexyl)-benzol
Trifluormethxoy-4-(trans-4-decylcyclohexyl)-benzol

12. Trifluormethylphenylether nach Anspruch 6, ausgewählt aus:
Trifluormethoxy-4-[trans-4-(trans-4-propylcyclohexyl)cyclohexyl]-benzol
Trifluormethoxy-4-[trans-4-(trans-4-ethylcyclohexyl)cyclohexyl]-benzol
Trifluormethoxy-4-[trans-4-(trans-4-butylcyclohexyl)cyclohexyl]-benzol
Trifluormethoxy-4-[trans-4-(trans-4-pentylcyclohexyl)cyclohexyl]-benzol
Trifluormethoxy-4-[trans-4-(trans-4-hexylcyclohexyl)cyclohexyl]-benzol
Trifluormethoxy-4-[trans-4-(trans-4-heptylcyclohexyl)cyclohexyl]-benzol
Trifluormethoxy-4-[trans-4-(trans-4-octylcyclohexyl)cyclohexyl]-benzol
Trifluormethoxy-4-[trans-4-(trans-4-nonylcyclohexyl)cyclohexyl]-benzol
Trifluormethoxy-4-[trans-4-(trans-4-decylcyclohexyl)cyclohexyl]-benzol.

13. Trifluormethylphenylether nach Anspruch 8, ausgewählt aus:
1-(4-Trifluormethoxyphenyl)-2-[trans-4-(trans-4-propylcylcohexyl)-cyclohexyl]-ethan
1-(4-Trifluormethoxyphenyl)-2-[trans-4-(trans-4-ethylcyclohexyl)cyclohexyl]-ethan
1-(4-Trifluormethoxyphenyl)-2-[trans-4-(trans-4-butylcyclohexyl)cyclohexyl]-ethan
1-(4-Trifluormethoxyphenyl)-2-[trans-4-(trans-4-pentylcyclohexyl)cyclohexyl]-ethan
1-(4-Trifluormethoxyphenyl)-2-[trans-4-(trans-4-hexylcyclohexyl)cyclohexyl]-ethan
1-(4-Trifluormethoxyphenyl)-2-[trans-4-(trans-4-heptylcyclohexyl)cyclohexyl]-ethan.

14. Verwendung der Verbindungen der Formel I nach Anspruch 1 als Komponenten flüssigkristalliner Phasen.

15. Flüssigkristalline Phase mit mindestens zwei flüssigkristallinen Komponenten, dadurch gekennzeichnet, daß sie mindestens eine Verbindung der Formel I nach Anspruch 1 enthält.

16. Flüssigkristall-Anzeigeelement, dadurch gekennzeichnet, daß es eine flüssigkristalline Phase nach Anspruch 15 enthält.

17. Elektrooptisches Anzeigeelement, dadurch gekennzeichnet, daß es als Dielektrikum eine flüssigkristalline Phase nach Anspruch 15 enthält.

## Claims

1. Substituted phenyl trifluoromethyl ethers of the formula I
R¹-(A¹-Z¹)ₙ-A²-Z²-A³-OCF₃ I
in which
R¹ is H or alkyl having 1-18 C atoms in which one or more CH₂ groups may also be replaced by -E-, -O-, -S- and/or -CO-, where two heteroatoms are not linked directly to one another, or perfluoroalkyl having 1-18 C atoms in which one or more CF₂ groups may also be replaced by -CH₂-, -E-, -O-, -S- and/or -CO-, where two heteroatoms are not linked directly to one another,
E is 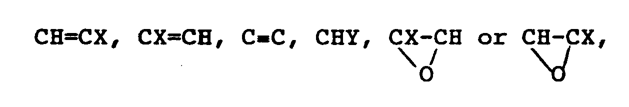
X is Y, CH₃ or H,
Y is CN, NCS, NCO or halogen,
A¹ and A², independently of one another, are each 1,4-cyclohexylene, in which one or two non-adjacent CH₂ groups may also be replaced by -O- and/or S atoms, 1,4-cyclohexenylene, 1,4-bicyclo(2.2.2)octylene, piperidine-1,4-diyl or 1,4-phenylene, in which one or more CH groups may be replaced by N, which is unsubstituted or monosubstituted or Polysubstituted by halogen atoms and/or CN and/or CH₃ groups,
Z¹ is -O-CO-, -CH₂O-, -OCH₂-, -CH₂CH₂-, -CH=CH- or a single bond,
Z² is -CO-O-, -CH₂O-, -OCH₂-, -CH₂CH₂-, -CH=CH- or a single bond,
A³ is a 1,4-phenylene group which is unsubstituted or monosubstituted or polysubstituted by halogen atoms and/or CN and/or CH₃ groups,
and
n is 0, 1 or 2,
with the provisos that
a) Z² is -CH₂O-, -OCH₂-, -CH₂CH₂-, -CH=CH- or a single bond in the case where n = 0 or 1, R¹ = alkoxy and A¹ = A² = A³ = unsubstituted 1,4-phenylene, and
b) 4-phenyltrifluoromethoxybenzene is excepted.

2. Phenyl trifluoromethyl ethers according to Claim 1, selected from the sub-formulae Ia and Ib:
R¹-A²-A³-OCF₃ Ia
R¹-A²-Z²-A³-OCF₃ Ib
in which R¹, A², A³ and Z² are as defined in Claim 1.

3. Phenyl trifluoromethyl ethers according to Claim 1, selected from the sub-formulae Ic to If:
R¹-A¹-A²-A³-OCF₃ Ic
R¹-A¹-Z¹-A²-Z²-A³-OCF₃ Id
R¹-A¹-Z¹-A²-A³-OCF₃ Ie
R¹-A¹-A²-Z²-A³-OCF₃ If
in which R¹, A¹, A², A³, Z¹ and Z² are as defined in Claim 1.

4. Phenyl trifluoromethyl ethers according to Claim 1, selected from the sub-formulae Ig to In:
R¹-A¹-A¹-A²-A³-OCF₃ Ig
R¹-A¹-Z¹-A¹-A²-A³-OCF₃ Ih
R¹-A¹-A¹-Z¹-A²-A³-OCF₃ Ii
R¹-A¹-A¹-A²-Z²-A³-OCF₃ Ij
R¹-A¹-Z¹-A¹-Z¹-A²-A³-OCF₃ Ik
R¹-A¹-Z¹-A¹-A²-Z²-A³-OCF₃ Il
R¹-A¹-A¹-Z¹-A²-Z²-A³-OCF₃ Im
R¹-A¹-Z¹-A¹-Z¹-A²-Z²-A³-OCF₃ In
in which R¹, A¹, A², A³, Z¹ and Z² are as defined in Claim 1.

5. Phenyl trifluoromethyl ethers according to Claim 2, selected from the sub-formulae Iaa to Iaf:
R¹-Phe-Phe-OCF₃ Iaa
R¹-Dio-Phe-OCF₃ Iab
R¹-Pyr-Phe-OCF₃ Iac
R¹-Pyd-Phe-OCF₃ Iad
R¹-Cyc-Phe-OCF₃ Iae
R¹-Che-Phe-OCF₃ Iaf
in which
R¹ is as defined in Claim 1,
Phe is a 1,4-phenylene group which is unsubstituted or substituted by one or more halogen atoms and/or CH₃ groups and/or CN groups,
Dio is a 1,3-dioxane-2,5-diyl group,
Pyr is a pyrimidine-2,5-diyl group,
Pyd is a pyridine-2,5-diyl group,
Cyc is a 1,4-cyclohexylene group which is unsubstituted or substituted by one or more halogen atoms and/or CH₃ groups and/or CN groups, and
Che is a 1,4-cyclohexenylene group.

6. Phenyl trifluoromethyl ethers according to Claim 3, selected from the sub-formulae Ica to Ici
R¹-Phe-Phe-Phe-OCF₃ Ica
R¹-Phe-Dio-Phe-OCF₃ Icb
R¹-Cyc-Cyc-Phe-OCF₃ Icc
R¹-Pyd-Phe-Phe-OCF₃ Icd
R¹-Pyr-Phe-Phe-OCF₃ Ice
R¹-Cyc-Phe-Phe-OCF₃ Icf
R¹-Dio-Phe-Phe-OCF₃ Icg
R¹-Che-Phe-Phe-OCF₃ Ich
R¹-Phe-Che-Phe-OCF₃ Ici
in which R¹ is as defined in Claim 1, and Phe, Dio, Cycl, Pyd, Pyr and Che are as defined in Claim 5.

7. Phenyl trifluoromethyl ethers according to Claim 3, selected from the sub-formulae Iea to Iei:
R¹-Pyr-Z¹-Phe-Phe-OCF₃ Iea
R¹-Dio-Z¹-Phe-Phe-OCF₃ Ieb
R¹-Cyc-Z¹-Phe-Phe-OCF₃ Iec
R¹-Phe-Z¹-Cyc-Phe-OCF₃ Ied
R¹-Cyc-Z¹-Cyc-Phe-OCF₃ Iee
R¹-Phe-Z¹-Dio-Phe-OCF₃ Ief
R¹-Pyd-Z¹-Phe-Phe-OCF₃ Ieg
R¹-Phe-Z¹-Pyr-Phe-OCF₃ Ieh
R¹-Phe-Z¹-Che-Phe-OCF₃ Iei
in which R¹ and Z¹ are as defined in Claim 1, and Phe, Dio, Cyc, Pyr and Che are as defined in Claim 5.

8. Phenyl trifluoromethyl ethers according to Claim 3, selected from the sub-formulae Ifa to Ifm:
R¹-Pyr-Phe-Z²-Phe-OCF₃ Ifa
R¹-Pyr-Phe-OCH₂-Phe-OCF₃ Ifb
R¹-Phe-Phe-Z²-Phe-OCF₃ Ifc
R¹-Cyc-Cyc-Z²-Phe-OCF₃ Ifd
R¹-Cyc-Cyc-CH₂CH₂-Phe-OCF₃ Ife
R¹-Pyd-Phe-Z²-Phe-OCF₃ Iff
R¹-Dio-Phe-Z²-Phe-OCF₃ Ifg
R¹-Phe-Cyc-Z²-Phe-OCF₃ Ifh
R¹-Phe-Pyd-Z²-Phe-OCF₃ Ifi
R¹-Che-Phe-Z²-Phe-OCF₃ Ifj
R¹-Phe-Che-Z²-Phe-OCF₃ Ifk
R¹-Pyr-Phe-CH₂CH₂-Phe-OCF₃ Ifl
R¹-Cyc-Phe-CH₂CH₂-Phe-OCF₃ Ifm
in which R¹ and Z¹ are as defined in Claim 1, and Phe, Dio, Cyc, Pyd, Pyr and Che are as defined in Claim 5.

9. Phenyl trifluoromethyl ethers according to one of Claims 1 to 4, characterised in that A³ is a 1,4-phenylene group which is unsubsituted or substituted by one or two F atoms.

10. Phenyl trifluoromethyl ethers according to Claim 9, selected from the formulae 1 to 13:

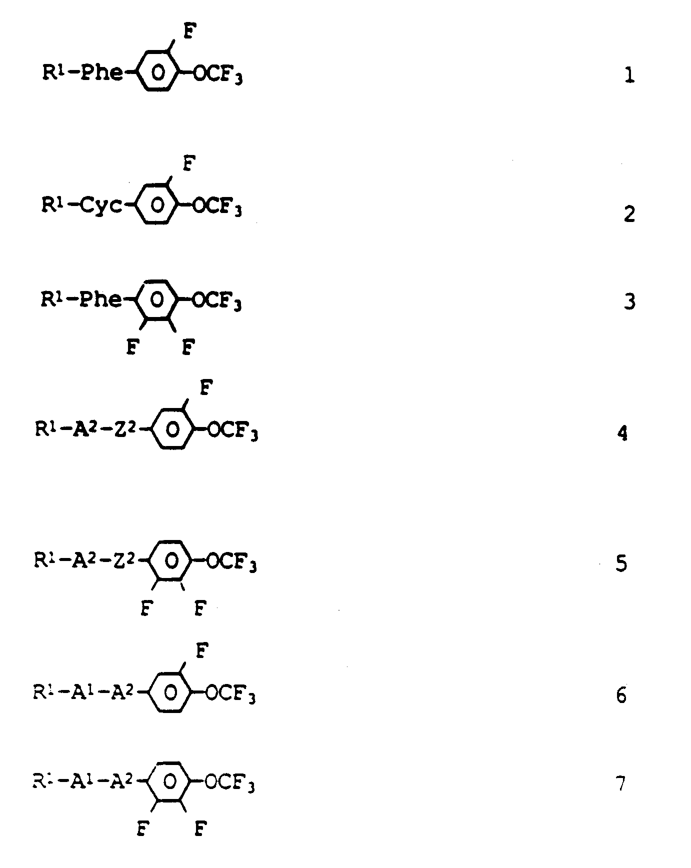

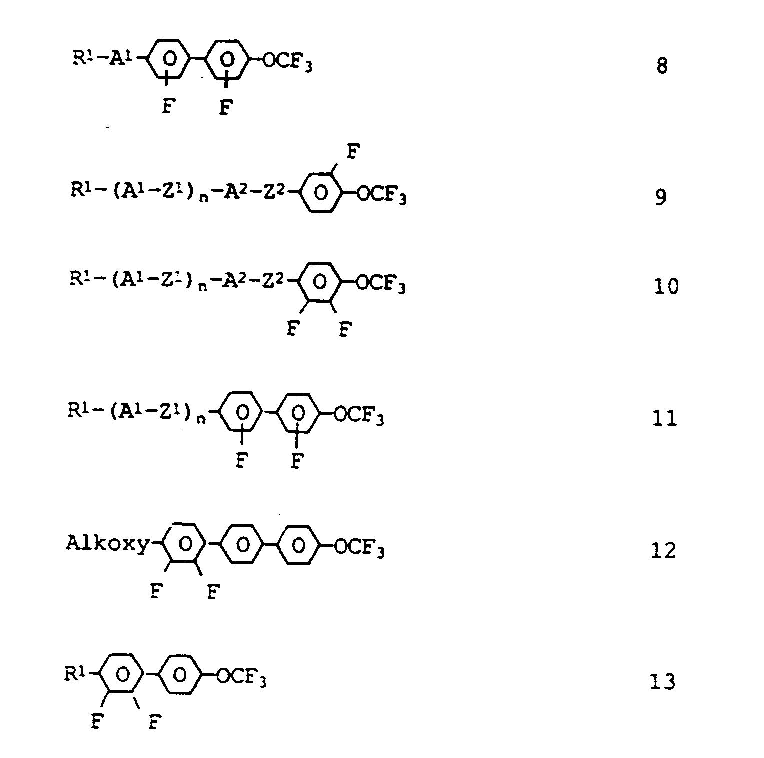

11. Trifluoromethyl phenyl ethers according to Claim 5, selected from:
Trifluoromethoxy-4-(trans-ethylcyclohexyl)benzene
Trifluoromethoxy-4-(trans-4-propylcyclohexyl)benzene
Trifluoromethoxy-4-(trans-4-butylcyclohexyl)benzene
Trifluoromethoxy-4-(trans-4-pentylcyclohexyl)benzene
Trifluoromethoxy-4-(trans-4-hexylcyclohexyl)benzene
Trifluoromethoxy-4-(trans-4-heptylcyclohexyl)benzene
Trifluoromethoxy-4-(trans-4-octylcyclohexyl)benzene
Trifluoromethoxy-4-(trans-4-nonylcyclohexyl)benzene
Trifluoromethoxy-4-(trans-4-decylcyclohexyl)benzene

12. Trifluoromethyl phenyl ethers according to Claim 6, selected from
Trifluoromethoxy-4-[trans-4-(trans-4-propylcyclohexyl)cyclohexyl]benzene
Trifluoromethoxy-4-[trans-4-(trans-4-ethylcyclohexyl)cyclohexyl]benzene
Trifluoromethoxy-4-[trans-4-(trans-4-butylcyclohexyl)cyclohexyl]benzene
Trifluoromethoxy-4-[trans-4-(trans-4-pentylcyclohexyl)cyclohexyl]benzene
Trifluoromethoxy-4-[trans-4-(trans-4-hexylcyclohexyl)cyclohexyl]benzene
Trifluoromethoxy-4-[trans-4-(trans-4-heptylcyclohexyl)cyclohexyl]benzene
Trifluoromethoxy-4-[trans-4-(trans-4-octylcyclohexyl)cyclohexyl]benzene
Trifluoromethoxy-4-[trans-4-(trans-4-nonylcyclohexyl)cyclohexyl]benzene
Trifluoromethoxy-4-[trans-4-(trans-4-decylcyclohexyl)cyclohexyl]benzene

13. Trifluoromethyl phenyl ethers according to Claim 8, selected from:
1-(4-Trifluoromethoxyphenyl)-2-[trans-4-(trans-4-propylcyclohexyl)cyclohexyl]ethane
1-(4-Trifluoromethoxyphenyl)-2-[trans-4-(trans-4-ethylcyclohexyl)cyclohexyl]ethane
1-(4-Trifluoromethoxyphenyl)-2-[trans-4-(trans-4-butylcyclohexyl)cyclohexyl]ethane
1-(4-Trifluoromethoxyphenyl)-2-[trans-4-(trans-4-pentylcyclohexyl)cyclohexyl]ethane
1-(4-Trifluoromethoxyphenyl)-2-[trans-4-(trans-4-hexylcyclohexyl)cyclohexyl]ethane
1-(4-Trifluoromethoxyphenyl)-2-[trans-4-(trans-4-heptylcyclohexyl)cyclohexyl]ethane

14. Use of the compounds of the formula I according to Claim 1 as components of liquid-crystalline phases.

15. Liquid-crystalline phase containing at least two liquid-crystalline components, characterised in that it contains at least one compound of the formula I according to Claim 1.

16. Liquid-crystal display element, characterised in that it contains a liquid-crystalline phase according to Claim 15.

17. Electrooptical display element, characterised in that it contains, as dielectric, a liquid-crystalline phase according to Claim 15.

## Revendications

1. Phényltrifluorométhyléthers substitués de formule I
R¹-(A¹-Z¹)ₙ-A²-Z²-A³-OCF₃ I
dans laquelle
R¹ représente H ou bien un groupe alkyle contenant de 1 à 18 atomes de carbone, dans lequel un ou plusieurs groupes CH₂ peuvent également être remplacés par -E-, -O-, -S- et/ou -CO-, deux hétéro-atomes n'étant pas reliés directement l'un à l'autre, ou encore un groupe perfluoroalkyle contenant de 1 à 18 atomes de carbone, dans lequel un ou plusieurs groupes CF₂ peuvent également être remplacés par -CH₂-, -E-, -O-, -S- et/ou -CO-, deux hétéro-atomes n'étant pas reliés directement l'un à l'autre,
E représente 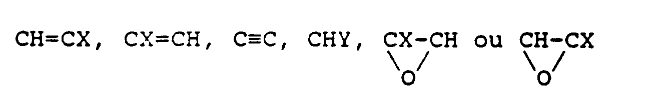
X représente Y, CH₃, H,
Y représente CN, NCS, NCO, un atome d'halogène,
A¹ et A² représentent chacun, indépendamment l'un de l'autre, un groupe 1,4-phénylène non substitué ou substitué une ou plusieurs fois par des atomes d'halogène et/ou par des groupes CN et/ou CH₃, un ou plusieurs groupes CH pouvant également être remplacés par N, un groupe 1,4-cyclohexylène dans lequel un ou deux groupes CH₂ non voisins peuvent également être remplacés par des atomes -O- et/ou S, un groupe 1,4-cyclohexénylène, un groupe 1,4-bicyclo(2.2.2)octylène ou un groupe pipéridine-1,4-diyle,
Z¹ représente -O-CO-, -CH₂O-, -OCH₂-, -CH₂CH₂-, -CH=CH- ou une liaison simple,
Z² représente -CO-O-, -CH₂O-, -OCH₂-, -CH₂CH₂-, -CH=CH- ou une liaison simple,
A³ représente un groupe 1,4-phénylène non substitué ou substitué une ou plusieurs fois par des atomes d'halogène et/ou par des groupes CN et/ou CH₃, et
n est égal à 0, 1 ou 2,
avec les mesures que :
a) au cas où n est égal à 0 ou 1, R¹ = un groupe alcoxy et A¹ = A²= A³= un groupe 1,4-phénylène non substitué, Z² représente -CH₂O-, -OCH₂-, -CH₂CH₂-, -CH=CH- ou une liaison simple, et
b) le groupe 4-phényltrifluorométhoxybenzène est exclus.

2. Phényltrifluorométhyléthers selon la revendication 1, choisis parmi les formules partielles Ia et Ib :
R¹-A²-A³-OCF₃ Ia
R¹-A²-Z²-A³-OCF₃ Ib
dans lesquelles R¹, A², A³ et Z² ont la signification indiquée à la revendication 1.

3. Phényltrifluorométhyléthers selon la revendication 1, choisis parmi les formules partielles Ic à If :
R¹-A¹-A²-A³-OCF₃ Ic
R¹-A¹-Z¹-A²-Z²-A³-OCF₃ Id
R¹-A¹-Z¹-A²-A³-OCF₃ Ie
R¹-A¹-A²-Z²-A³-OCF₃ If
dans lesquelles R¹, A¹, A², A³, Z¹ et Z² ont la signification indiquée à la revendication 1.

4. Phényltrifluorométhyléthers selon la revendication 1, choisis parmi les formules partielles Ig à In :
R¹-A¹-A¹-A²-A³-OCF₃ Ig
R¹-A¹-Z¹-A¹-A²-A³-OCF₃ Ih
R¹-A¹-A¹-Z¹-A²-A³-OCF₃ Ii
R¹-A¹-A¹-A²-Z²-A³-OCF₃ Ij
R¹-A¹-Z¹-A¹-Z¹-A²-A³-OCF₃ Ik
R¹-A¹-Z¹-A¹-A²-Z²-A³-OCF₃ Il
R¹-A¹-A¹-Z¹-A²-Z²-A³-OCF₃ Im
R¹-A¹-Z¹-A¹-Z¹-A²-Z²-A³-OCF₃ In
dans lesquelles R¹, A¹, A², A³, Z¹ et Z² ont la signification indiquée à la revendication 1.

5. Phényltrifluorométhyléthers selon la revendication 2, choisis parmi les formules partielles Iaa à Iaf :
R¹-Phe-Phe-OCF₃ Iaa
R¹-Dio-Phe-OCF₃ Iab
R¹-Pyr-Phe-OCF₃ Iac
R¹-Pyd-Phe-OCF₃ Iad
R¹-Cyc-Phe-OCF₃ Iae
R¹-Che-Phe-OCF₃ Iaf
R¹ a la signification indiquée dans la revendication 1,
Phe désigne un groupe 1,4-phénylène non substitué ou substitué par un ou plusieurs atomes d'halogène et/ou par des groupes CH₃ et/ou par des groupes CN,
Dio désigne un groupe 1,3-dioxanne-2,5-diyle,
Pyr désigne un groupe pyrimidine-2,5-diyle,
Pyd désigne un groupe pyridine-2,5-diyle,
Cyc désigne un groupe 1,4-cyclohexylène non substitué ou substitué par un ou plusieurs atomes d'halogène et/ou par des groupes CH₃ et/ou par des groupes CN,
Che désigne un groupe 1,4-cyclohexénylène.

6. Phényltrifluorométhyléthers selon la revendication 3, choisis parmi les formules partielles Ica à Ici :
R¹-Phe-Phe-Phe-OCF₃ Ica
R¹-Phe-Dio-Phe-OCF₃ Icb
R¹-Cyc-Cyc-Phe-OCF₃ Icc
R¹-Pyd-Phe-Phe-OCF₃ Icd
R¹-Pyr-Phe-Phe-OCF₃ Ice
R¹-Cyc-Phe-Phe-OCF₃ Icf
R¹-Dio-Phe-Phe-OCF₃ Icg
R¹-Che-Phe-Phe-OCF₃ Ich
R¹-Phe-Che-Phe-OCF₃ Ici
dans lesquelles R¹ a la signification indiquée à la revendication 1 et Phe, Dio, Cyc, Pyd, Pyr et Che ont la signification indiquée à la revendication 5.

7. Phényltrifluorométhyléthers selon la revendication 3, choisis parmi les formules partielles Iea à Iei :
R¹-Pyr-Z¹-Phe-Phe-OCF₃ Iea
R¹-Dio-Z¹-Phe-Phe-OCF₃ Ieb
R¹-Cyc-Z¹-Phe-Phe-OCF₃ Iec
R¹-Phe-Z¹-Cyc-Phe-OCF₃ Ied
R¹-Cyc-Z¹-Cyc-Phe-OCF₃ Iee
R¹-Phe-Z¹-Dio-Phe-OCF₃ Ief
R¹-Pyd-Z¹-Phe-Phe-OCF₃ Ieg
R¹-Phe-Z¹-Pyr-Phe-OCF₃ Ieh
R¹-Phe-Z¹-Che-Phe-OCF₃ Iei
dans lesquelles R¹ et Z¹ ont la signification indiquée à la revendication 1 et Phe, Dio, Cyc, Pyr et Che ont la signification indiquée à la revendication 5.

8. Phényltrifluorométhyléthers selon la revendication 3, choisis parmi les formules partielles Ifa à Ifm :
R¹-Pyr-Phe-Z²-Phe-OCF₃ Ifa
R¹-Pyr-Phe-OCH₂-Phe-OCF₃ Ifb
R¹-Phe-Phe-Z²-Phe-OCF₃ Ifc
R¹-Cyc-Cyc-Z²-Phe-OCF₃ Ifd
R¹-Cyc-Cyc-CH₂CH₂-Phe-OCF₃ Ife
R¹-Pyd-Phe-Z²-Phe-OCF₃ Iff
R¹-Dio-Phe-Z²-Phe-OCF₃ Ifg
R¹-Phe-Cyc-Z²-Phe-OCF₃ Ifh
R¹-Phe-Pyd-Z²-Phe-OCF₃ Ifi
R¹-Che-Phe-Z²-Phe-OCF₃ Ifj
R¹-Phe-Che-Z²-Phe-OCF₃ Ifk
R¹-Pyr-Phe-CH₂CH₂-Phe-OCF₃ Ifl
R¹-Cyc-Phe-CH₂CH₂-Phe-OCF₃ Ifm
dans lesquelles R¹ et Z¹ ont la signification indiquée à la revendication 1 et Phe, Dio, Cyc, Pyd, Pyr et Che ont la signification indiquée à la revendication 5.

9. Phényltrifluorométhyléthers selon l'une quelconque des revendications 1 à 4, caractérisés en ce que A³ représente un groupe 1,4-phénylène non substitué ou substitué par un ou deux atomes de F.

10. Phényltrifluorométhyléthers selon la revendication 9, choisis parmi les formules 1 à 13 :

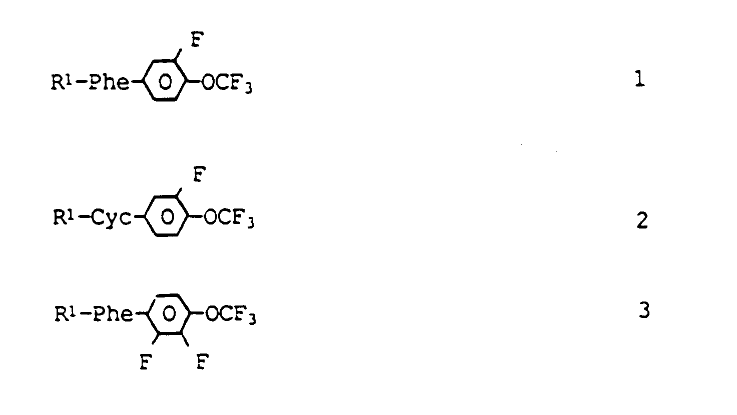

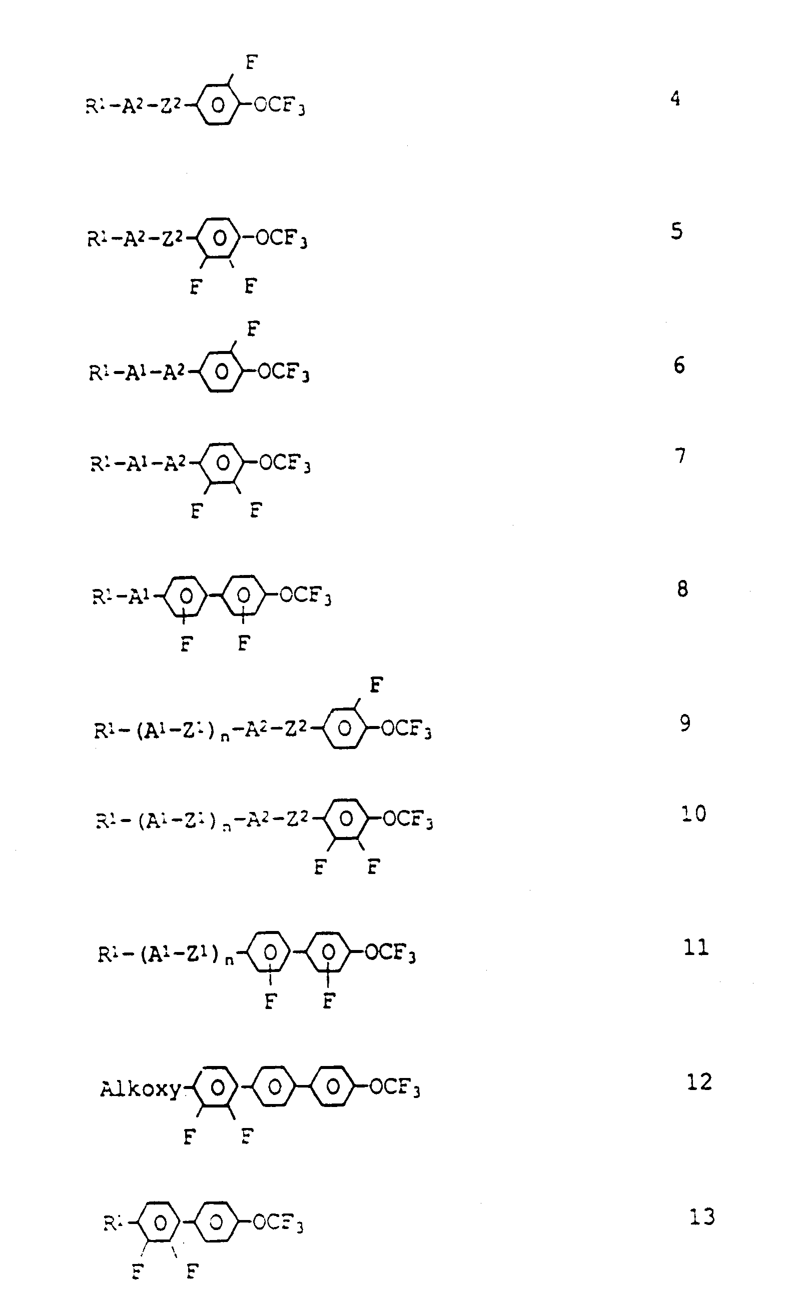

11. Phényltrifluorométhyléthers selon la revendication 5, choisis parmi :
le trifluorométhoxy-4-(trans-4-éthylcyclohexyl)-benzène
le trifluorométhoxy-4-(trans-4-propylcyclohexyl)-benzène
le trifluorométhoxy-4-(trans-4-butylcyclohexyl)-benzène
le trifluorométhoxy-4-(trans-4-pentylcyclohexyl)-benzène
le trifluorométhoxy-4-(trans-4-hexylcyclohexyl)-benzène
le trifluorométhoxy-4-(trans-4-heptylcyclohexyl)-benzène
le trifluorométhoxy-4-(trans-4-octylcyclohexyl)-benzène
le trifluorométhoxy-4-(trans-4-nonylcyclohexyl)-benzène
le trifluorométhoxy-4-(trans-4-décylcyclohexyl)-benzène

12. Phényltrifluorométhyléthers selon la revendication 6, choisis parmi :
le trifluorométhoxy-4-[trans-4-(trans-4-propylcyclohexyl)cyclohexyl]-benzène
le trifluorométhoxy-4-[trans-4-(trans-4-éthylcyclohexyl)cyclohexyl]-benzène
trifluorométhoxy-4-[trans-4-(trans-4-butylcyclohexyl)cyclohexyl]-benzène
trifluorométhoxy-4-[trans-4-(trans-4-pentylcyclohexyl)cyclohexyl]-benzène
trifluorométhoxy-4-[trans-4-(trans-4-hexylcyclohexyl)cyclohexyl]-benzène
trifluorométhoxy-4-[trans-4-(trans-4-heptylcyclohexyl)cyclohexyl]-benzène
trifluorométhoxy-4-[trans-4-(trans-4-octylcyclohexyl)cyclohexyl)-benzène
trifluorométhoxy-4-[trans-4-(trans-4-nonylcyclohexyl)cyclohexyl]-benzène
trifluorométhoxy-4-[trans-4-(trans-4-décylcyclohexyl)cyclohexyl]-benzène

13. Phényltrifluorométhyléthers selon la revendication 8, choisis parmi
le 1-(4-trifluorométhoxyphényl)-2-[trans-4-(trans-4-propylcyclohexyl)cyclohexyl)-éthane
le 1-(4-trifluorométhoxyphényl)-2-[trans-4-(trans-4-éthylcyclohexyl)cyclohexyl]-éthane
le 1-(4-trifluorométhoxyphényl)-2-[trans-4-(trans-4-butylcyclohexyl)cyclohexyl]-éthane
le 1-(4-trifluorométhoxyphényl)-2-[trans-4-(trans-4-pentylcyclohexyl)cyclohexyl]-éthane
le 1-(4-trifluorométhoxyphényl)-2-[trans-4-(trans-4-hexylcyclohexyl)cyclohexyl]-éthane
le 1-(4-trifluorométhoxyphényl)-2-[trans-4-(trans-4-heptylcyclohexyl)cyclohexyl]-éthane

14. Utilisation des composés de formule I selon la revendication 1, comme composants de phases à cristaux liquides.

15. Phase à cristaux liquides contenant au moins deux composants à cristaux liquides, caractérisée en ce qu'elle contient au moins un composé de formule I selon la revendication 1.

16. Elément d'affichage à cristaux liquides, caractérisé en ce qu'il contient une phase à cristaux liquides selon la revendication 15.

17. Elément d'affichage électro-optique caractérisé en ce qu'il contient, comme diélectrique, une phase à cristaux liquides selon la revendication 15.
